(19) Europäisches Patentamt
European Patent Office
Office européen des brevets

(11) EP 4 692 359 A1

(12) EUROPEAN PATENT APPLICATION
published in accordance with Art. 153(4) EPC

(43) Date of publication:
**11.02.2026 Bulletin 2026/07**

(21) Application number: **24780517.9**

(22) Date of filing: **27.03.2024**

(51) International Patent Classification (IPC):
***C12N 15/86*** (2006.01) ***C12N 5/10*** (2006.01)
***C12N 7/00*** (2006.01) ***C12N 7/02*** (2006.01)
***C12N 13/00*** (2006.01) ***C12N 15/63*** (2006.01)
***C12N 15/87*** (2006.01) ***C12N 15/864*** (2006.01)

(52) Cooperative Patent Classification (CPC):
**C12N 5/10; C12N 7/00; C12N 7/02; C12N 13/00; C12N 15/63; C12N 15/86; C12N 15/864; C12N 15/87**

(86) International application number:
**PCT/JP2024/012393**

(87) International publication number:
**WO 2024/204415 (03.10.2024 Gazette 2024/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.03.2023 JP 2023050311**
**19.03.2024 JP 2024042987**

(71) Applicant: **FUJIFILM Corporation**
**Tokyo 106-8620 (JP)**

(72) Inventors:
- **YAKUSHIJI, Takashi**
**Ashigarakami-gun, Kanagawa 258-8577 (JP)**
- **EDO, Michiko**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) VIRUS PRODUCTION METHOD

(57) An object of the present invention is to provide a virus production method capable of improving an amount of virus production. According to the present invention, there is provided a virus production method including a nucleic acid introduction step of introducing a nucleic acid into cells via electroporation to obtain cells into which the nucleic acid has been introduced; and a culture step of culturing the cells into which the nucleic acid has been introduced, in which a CNET product defined in the present specification in the electroporation is $1 \times 10^4$ or more and $1 \times 10^7$ or less.

EP 4 692 359 A1

## Description

### Technical Field

The present invention relates to a virus production method including introducing a nucleic acid into cells via electroporation.

### Background Art

A gene therapy method has been developed in which a recombinant adeno-associated virus (AAV) vector into which a therapeutic gene has been incorporated is administered to a patient for the purpose of treatment of an intractable disease such as a genetic disease, cancer, or the like. As a method of producing an AAV vector, a method has been established in which a complex is formed between a plasmid DNA having a negative charge and polyethyleneimine (PEI) as a cationic polymer to neutralize the charge of the plasmid DNA, a plasmid DNA encoding proteins necessary for AAV production is introduced into cells by endocytosis, and AAV is produced in the cells.

In addition, a physical introduction method is known as a method of introducing a gene into cells without using a virus vector and without using a chemical reagent (for example, polyethyleneimine, lipid nanoparticles, and the like). The physical introduction method includes electroporation, which makes use of cell membrane perforation by electric field application and the principle of electrophoresis. In electroporation, the gene introduction into cells is completed at the moment of pulse application (electrophoresis), and thus it is not necessary to control the stirring of the plasmid and the cells for a long time. In addition, for electroporation, a method of processing a large amount of cells by a flow method has also been studied.

Patent Document 1 describes that AAV is produced by introducing a recombinant AAV genome and REP mRNA into eukaryotic cells via electroporation. In Patent Document 1, specific conditions for electroporation are not described. In addition, a virus vector is used for introducing the helper function, and helper genes are introduced via viral infection.

### Prior Art Documents

### Patent Documents

Patent Document 1: JP2020-511153A

### Summary of Invention

### Object to be solved by the invention

Since the conventional target diseases have been localized diseases, there has been little need for large-scale production of AAV. In the future, it is assumed that there will be an increasing need for the treatment of systemic diseases, and the improvement of the productivity of AAV is an issue.

An object to be achieved of the present invention is to provide a virus production method capable of improving an amount of virus production.

### Means for solving the object

As a result of intensive studies to achieve the above object, the inventors of the present invention have found that, in a case of introducing a nucleic acid into cells via electroporation to obtain cells into which the nucleic acid has been introduced, the amount of virus production can be improved by setting the CNET product as defined in the present specification in the electroporation to $1 \times 10^4$ or more and $1 \times 10^7$ or less. The present invention has been completed based on the above findings.

According to an aspect of the present invention, the following invention is provided.

<1> A virus production method comprising: a nucleic acid introduction step of introducing a nucleic acid into cells via electroporation to obtain cells into which the nucleic acid has been introduced; and a culture step of culturing the cells into which the nucleic acid has been introduced, in which a CNET product represented by the following formula in the electroporation is $1 \times 10^4$ or more and $1 \times 10^7$ or less,

$$\sum_{k=1}^{n} C|N_k E_k T_k| \quad \text{or} \quad \sum_{k=1}^{n} C|N_k E_k T_k| + \sum_{l=1}^{m} C|N_l E_l T_l|$$

in the formulae, C represents a nucleic acid concentration in terms of μg/mL,
$N_k$ represents the number of pulses, and $N_k$ is an integer of 1 or more,
$E_k$ represents a pulsed electric field in terms of V/cm, and $E_k$ is 500 V/cm or more and 2,000 V/cm or less,
$T_k$ represents a pulse duration in terms of ms,
$N_l$ represents the number of pulses, and $N_l$ is an integer of 1 or more,
$E_l$ represents a pulsed electric field in terms of V/cm, and $E_l$ is 50 V/cm or more and less than 500 V/cm,
$T_l$ represents a pulse duration in terms of ms,
k represents an integer from 1 to n,
l represents an integer from 1 to m,
n represents an integer of 1 or more, and
m represents an integer of 1 or more.

<2> The virus production method according to <1>, in which the introduction of the nucleic acid is performed only via the electroporation.
<3> The virus production method according to <1> or <2>, in which the CNET product is $1 \times 10^5$ or more and $1 \times 10^6$ or less.
<4> The virus production method according to any one of <1> to <3>, in which the CNET product is represented by

$$\sum_{k=1}^{n} C|N_k E_k T_k| + \sum_{l=1}^{m} C|N_l E_l T_l|$$

, and $\sum_{k=1}^{n} C|N_k E_k T_k| < \sum_{l=1}^{m} C|N_l E_l T_l|$ is satisfied.

<5> The virus production method according to any one of <1> to <4>, in which the nucleic acid concentration C is 10 to 500 μg/mL.
<6> The virus production method according to any one of <1> to <5>, in which in a virus produced in the culture step, a ratio of an amount of capsids containing a full-length gene to a total amount of capsids is 30% or more.
<7> The virus production method according to any one of <1> to <6>, in which the nucleic acid is introduced into the cells in an absence of a transfection reagent.
<8> The virus production method according to any one of <1> to <7>, in which the cells are in a suspension in the nucleic acid introduction step, and the suspension has a conductivity of 5 to 20 mS/cm.
<9> The virus production method according to any one of <1> to <8>, in which the cells are in a suspension in the nucleic acid introduction step and the culture step, and the suspension is a culture medium.
<10> The virus production method according to any one of <1> to <9>, in which after the nucleic acid introduction step, an additional nucleic acid introduction is not performed.
<11> The virus production method according to any one of <1> to <10>, in which a virus is an adeno-associated virus.
<12> The virus production method according to any one of <1> to <11>, in which the nucleic acid contains one or more of an adeno-associated virus gene and a virus helper gene.
<13> The virus production method according to any one of <1> to <12>, in which the nucleic acid contains at least one or more virus helper genes.
<14> The virus production method according to any one of <1> to <13>, in which the nucleic acid contains four or more genes selected from the group consisting of a gene for treatment or prevention, a Rep gene, a Cap gene, an E2 gene, an E4 gene, and a VA-RNA1 gene.
<15> The virus production method according to any one of <1> to <14>, in which the nucleic acid is introduced into the cells using a plurality of plasmids.
<16> The virus production method according to any one of <1> to <15>, in which the electroporation is flow electroporation.
<17> The virus production method according to any one of <1> to <16>, in which a cell concentration during the nucleic acid introduction is $10 \times 10^6$ cells/mL to $200 \times 10^6$ cells/mL.
<18> The virus production method according to any one of <1> to <17>, in which feeding of liquid is performed aseptically in the nucleic acid introduction step and the culture step.
<19> The virus production method according to any one of <1> to <18>, in which the cells are animal cells.
<20> The virus production method according to any one of <1> to <19>, in which the cells are HEK cells.

<21> The virus production method according to any one of <1> to <20>, further including recovering a produced virus.

**Effect of the invention**

According to the virus production method of the present invention, the amount of virus production can be improved.

**BRIEF DESCRIPTION OF THE DRAWINGS**

[FIG. 1] FIG. 1 is a top view of an electroporation device.
[FIG. 2] FIG. 2 is a cross-sectional view of the electroporation device.
[FIG. 3] FIG. 3 shows an example of a pulse waveform of electroporation.
[FIG. 4] FIG. 4 shows an example of a pulse waveform of electroporation.
[FIG. 5] FIG. 5 shows an example of a pulse waveform of electroporation.
[FIG. 6] FIG. 6 shows an example of a pulse waveform of electroporation.
[FIG. 7] FIG. 7 is a schematic view showing perfusion culture.
[FIG. 8] FIG. 8 is a schematic view showing pAAV-GFP, pAAV-RC5, and pHelper.
[FIG. 9] FIG. 9 is a schematic view showing a continuous production apparatus for AAV in which a perfusion culture device are connected to a flow electroporation device.

**Embodiments for carrying out the invention**

Hereinafter, an example of an embodiment of the present disclosure will be described. However, the present disclosure is not limited to the following embodiments, and can be implemented with appropriate changes within the scope of the object of the present disclosure. In the present specification, a numerical range indicated using "to" represents a range including numerical values described before and after "to" as a minimum value and a maximum value.
The present invention relates to a virus production method including a nucleic acid introduction step of introducing a nucleic acid into cells via electroporation to obtain cells into which the nucleic acid has been introduced; and a culture step of culturing the cells into which the nucleic acid has been introduced, in which a CNET product represented by the following formula in the electroporation is $1 \times 10^4$ or more and $1 \times 10^7$ or less.

$$\sum_{k=1}^{n} C|N_k E_k T_k| \quad \text{or} \quad \sum_{k=1}^{n} C|N_k E_k T_k| + \sum_{l=1}^{m} C|N_l E_l T_l|$$

In the formulae, C represents a nucleic acid concentration in terms of μg/mL,

$N_k$ represents the number of pulses, and $N_k$ is an integer of 1 or more,
$E_k$ represents a pulsed electric field in terms of V/cm, and $E_k$ is 500 V/cm or more and 2,000 V/cm or less,
$T_k$ represents a pulse duration in terms of ms,
$N_l$ represents the number of pulses, and $N_l$ is an integer of 1 or more,
$E_l$ represents a pulsed electric field in terms of V/cm, and $E_l$ is 50 V/cm or more and less than 500 V/cm,
$T_l$ represents a pulse duration in terms of ms,
k represents an integer from 1 to n,
l represents an integer from 1 to m,
n represents an integer of 1 or more, and
m represents an integer of 1 or more.

In the application to high-dose use for systemic diseases and the like, it is important to increase the full capsid rate from the viewpoints of reducing costs by improving the productivity of the gene therapy drug and reducing side effects in patients, and it is an object to improve the titer and the full capsid rate at the stage of the previous step before purification.
In the method using polyethyleneimine, the control of the plasmid/polyethyleneimine complex (aggregation over time and a decrease in introduction efficiency) and the control of stirring of cells and the plasmid/polyethyleneimine complex in the reactor are complicated, and it is difficult to scale up and achieve high cell concentration. In addition, there is also an issue with the cytotoxicity of polyethyleneimine itself. In gene introduction via electroporation, it is necessary to control the amount of intracellularly introduced nucleic acids and to reduce cell damage, and there is no example in which electroporation has been put into practical use in the production of therapeutic viruses.

(1) Preculture of cells

The cells are not particularly limited, but are preferably animal cells, more preferably mammalian cells or insect cells, and still more preferably mammalian cells. Examples of the mammalian cell include a human cell, a mouse cell, a rat cell, a monkey cell, and a hamster cell, but the cells are not particularly limited. The human cell can be preferably used. Examples of the cell include mouse myeloma (NSO) cell lines, Chinese hamster ovary (CHO) cell lines, HT1080, H9, HepG2, MCF7, MDBK Jurkat, NIH3T3, PC12, a baby hamster kidney (BHK) cell, VERO, SP2/0, YB2/0, Y0, C127, an L cell, COS (for example, COS1 and COS7), QC1-3, a human embryo-derived kidney (HEK293) cell, VERO, PER. C6, HeLa, EB1, EB2, EB3, and oncolytic or hybridoma cell lines. The cell is preferably a HEK293 cell or a CHO cell, and more preferably a HEK293 cell. The HEK293 cells are cells into which an E1A gene and an E2B gene derived from an adenovirus are incorporated. The cells may be either adherent cells or suspension cells, but suspension cells are preferable to perform high-concentration large-scale culture in a reactor.

As a cell line having high cell proliferation properties and high AAV production ability per cell, for example, a suspension HEK293 cell line Viral Production Cells 2.0 (VPCs 2.0) manufactured by Thermo Fisher Scientific, Inc. can be used.

In addition, a group of gene coding sequences necessary for virus production may be incorporated into the genome of the cell in advance (packaging cell). Examples of the group of gene coding sequences necessary for AAV production include at least one or more selected from a target gene GoI (for example, a gene for treatment or prevention), REP, CAP, E2, E4, and VA-RNA, which will be described later.

As the culture medium that is used for culture, a culture medium that is used for culturing typical animal cells can be used. As the culture medium, a culture medium optimized for culturing HEK293 cells and the like based on PBS is available commercially by each company. For example, Balan-CD HEK293 medium manufactured by FUJIFILM Irvine Scientific (FISI), Expi293™ Expression Medium (manufactured by Thermo Fisher Scientific, Inc.), Gibco Viral Vector HEK Media Panel (manufactured by Thermo Fisher Scientific, Inc.), and the like can be used. In addition, CD OptiCHO (manufactured by Thermo Fisher Scientific, Inc.), Dulbecco's modified Eagle medium (DMEM), Eagle minimum essential medium (MEM), RPMI-1640 medium, RPMI-1641 medium, F-12K medium, Ham's F12 medium, Iscove's modified Dulbecco's medium (IMDM), McCoy's 5A medium, Leibovitz's L-15 medium, and EX-CELL (trade mark) 300 series (JRH Biosciences), CHO-S-SFMII (Invitrogen), CHO-SF (Sigma-Aldrich Co. LLC), CD-CHO (Invitrogen), IS CHO-V (FUJIFILM Irvine Scientific), PF-ACF-CHO (Sigma-Aldrich Co. LLC), and the like can be used. Alternatively, a homemade culture medium may be used. Additional component may be appropriately supplemented to the culture medium as necessary. Examples of the additional component include amino acids, salts, sugars (glucose and the like), vitamins, hormones, growth factors, lipids, trace elements, and the like, but the additional component is not particularly limited. The pH of the culture medium is 6 to 8, preferably 6.8 to 7.6, and more preferably 7.2 to 7.6.

An anti-foaming agent may be further added to the culture medium. As the anti-foaming agent, a silicone-based anti-foaming agent is preferable, and dimethicone is particularly preferable. The anti-foaming agent is preferably an anti-foaming component containing polydimethylsiloxane, and it is more preferably an anti-foaming component (simethicone) in which fine powder silica is contained in polydimethylsiloxane. The addition rate of the simethicone with respect to the amount of the culture solution is not particularly limited, but is preferably 5 mg/hr/L or less and more preferably 2 mg/hr/L or less.

A block copolymer of polyoxypropylene and polyoxyethylene may be added to the culture medium. The copolymer of polyoxypropylene and polyoxyethylene is preferably Poloxamer, where Poloxamer-188 is more preferable. The content of the block copolymer of polyoxypropylene and polyoxyethylene in the culture medium is not particularly limited; however, it is preferably 0.1% by mass or more and 2% by mass or less, more preferably 0.25% by mass or more and 2% by mass or less, and still more preferably 0.5% by mass or more and 2% by mass or less.

The method of preculture of cells is not particularly limited, and may be any of batch culture, fed-batch culture, perfusion culture, shaking culture, stirring culture, standing culture, or adhesion culture. From the viewpoints of large-scale treatment and high concentration, perfusion culture is preferable. In the perfusion culture, the removal of waste components by a filter and the supply of a fresh culture medium make it possible to perform proliferation at a high concentration by maintaining the quality (component concentration) of the suspension under conditions suitable for cell proliferation.

The temperature of the preculture of the cells is preferably 30°C to 40°C, more preferably 32°C to 37°C, and particularly preferably 36°C to 37°C. The $CO_2$ concentration is 5% to 10%.

The cell density is preferably $1 \times 10^6$ to $20 \times 10^6$ cells/mL in the batch culture, preferably $1 \times 10^6$ to $50 \times 10^6$ cells/mL in the fed-batch culture, and preferably $1 \times 10^6$ to $200 \times 10^6$ cells/mL in the perfusion culture (dynamic/continuous).

It is preferable to culture such that the cell density of the preculture is 10 × $10^6$ cells/mL or more and 200 × $10^6$ cells/mL or less. The cell density is more preferably 20 × $10^6$ cells/mL to 100 × $10^6$ cells/mL, and more preferably 40 × $10^6$ cells/mL to 80 × $10^6$ cells/mL.

The culture time of the preculture is preferably 12 hours or more.

In a case of perfusion culture, either a dynamic method or a continuous method may be used. In the dynamic method, cells are seeded at a low concentration, proliferative culture is performed, the total amount of the cell solution is collected after reaching a high concentration, and gene introduction is performed. In the continuous method, cells are seeded at a low concentration, proliferated culture is performed, a certain amount of cells are extracted (cell bleeding) after reaching a certain concentration, and gene introduction is performed. Fresh culture medium in the same amount as the extraction amount is supplemented on the reactor side to maintain the constant cell concentration in a reactor. The cell bleeding and the supply of the culture medium may be continuous or intermittent (for example, once a day at 10% to 50% or the like). The concentration is recovered by cell proliferation until the next cell bleeding. The above operation can be repeated, for example, for 1 day to 3 months. The perfusion ratio is not particularly limited; however, it is generally 0.3 vvd to 5.0 vvd preferably 0.5 vvd to 4.0 vvd. The vvd regarding the perfusion ratio means "volume of withdrawn culture solution/volume of culture solution in culture container/day"

Devices and culture methods for perfusion culture are known in the related art, and are described in WO2018/159847, WO2019/049843A, WO2019/181234A, WO2019/239780A, WO2020/003833A, WO2020/162125A, WO2021/187008A, WO2022/196710A, and WO2023/054556A, the contents of which are incorporated herein by reference. In addition, perfusion culture which will described later can also be used in the preculture. For example, the description of the "membrane separation treatment step of passing a cell suspension extracted from a culture tank through a separation membrane to separate the cell suspension into a cell-containing liquid and a permeated liquid" and the description related to FIG. 9, which will be described later, can be used in the preculture.

(2) Adjustment of cell concentration and exchange of culture medium

The cell concentration during the nucleic acid introduction is preferably 10 × $10^6$ cells/mL to 200 × $10^6$ cells/mL, more preferably 20 × $10^6$ cells/mL to 160 × $10^6$ cells/mL, and still more preferably 40 × $10^6$ cells/mL to 120 × $10^6$ cells/mL. The lower limit value is preferably 10 × $10^6$ cells/mL, more preferably 20 × $10^6$ cells/mL, still more preferably 40 × $10^6$ cells/mL, even still more preferably 80 × $10^6$ cells/mL, and yet even still more preferably 100 × $10^6$ cells/mL. The higher the cell concentration is, the more efficiently the nucleic acid can be introduced.

In the polyethyleneimine (PEI) method, a complex of a negatively charged plasmid DNA and a cationic polymer PEI is formed to neutralize the charge, and the PEI-plasmid complex can approach a negatively charged cell, and the complex is transferred into the cell by endocytosis. Then it leads to the endosome escape in the cell, the PEI release, and the gene transcription and translation reaction in the nucleus. The optimal size of the DNA/PEI complex for introduction into cells is about several hundred nm to several μm. The DNA/PEI complex tends to aggregate and precipitate over time in a liquid. The PEI method is a complicated reaction system influenced by the control of the optimal DNA/PEI complex state, the control of the collision with cells, the concentration of free nucleic acid (derived from cells) in the culture solution, the cell concentration, the reactor scale, the stirring speed, and the like. In the PEI method, it is necessary to optimize the culture conditions, the history, the cell concentration, the reactor scale, the concentration of the plasmid to be added according to stirring method, and the PEI concentration. In addition, the intracellular gene introduction step is complicated, and is performed in a range of several tens of minutes to several hours after the addition of the PEI complex to the cell culture solution. It is also necessary to compatible with culture conditions that can maintain cellular culture characteristics. In general, gene introduction is performed at a low cell concentration of about 1 × $10^6$ cells/mL.

In electroporation (EP), the perforation site is formed in cells by an electric pulse application, and a plasmid DNA that is negatively charged and repelled by the cell is brought close to and penetrated into the cells (the perforation site) by an electrophoresis action due to the electric pulse application, thereby realizing intracellular gene introduction. Therefore, unlike the PEI method, since the gene introduction in the EP method is completed at the time of pulse application, it is not necessary to control stirring and culture for a long time as in the PEI method, and the cell concentration can be temporarily changed freely only in the EP step as long as uniform dispersibility of the cells and the plasmid DNA can be maintained. In addition, since the introduction is based on the principle of electrophoresis, it is not necessary to individually optimize the concentration of added plasmid according to cell concentration or the state of the culture medium, and the same plasmid concentration condition can be applied in a wide range of cell concentrations. Therefore, from the viewpoint of improving the efficiency of using the plasmid and the viewpoint of reducing the amount of the treatment, it is preferable to set the cell concentration at the time of EP to be high.

The cell concentration may be increased as compared with the cell concentration at the time of the preculture, by cell concentration using centrifugation, continuous centrifugation, sonic agglomeration, acoustic electrophoresis, filtration (TFF, ATF), or the like. It is preferable that the concentration is performed to a cell concentration of 80 × $10^6$ cells/ml or more. The cell concentration is more preferably 100 × $10^6$ cells/ml or more and 120 × $10^6$ cells/ml or more.

In addition, in the same step, the culture medium of the suspension may be exchanged with a fresh culture medium, an electroporation dedicated buffer, or a culture medium optimized for subsequent culture.

As the conditions of the buffer at the time of EP, conditions suitable for typical cell culture (pH, pH buffering capacity, nutrients, salt concentration, and the like) can be used.

In the nucleic acid introduction step, it is preferable that the cells are in a suspension, and the conductivity of the suspension is 5 to 20 mS/cm. Provided that since it is possible to take measures to reduce heat generation under the conditions of electroporation, it is not essential that the conductivity in the suspension is 5 to 20 mS/cm.

In the electroporation, a membrane repair agent may be added, or a nutritional component may be added.

There are many similarities in the functions and components between the electroporation buffer and the culture medium for cell culture, and in some cases, the cell viability after EP, and the efficiency of gene introduction and expression may be increased by using a dedicated culture medium optimized for each cell, rather than a general-purpose EP buffer. In the HEK293 cell × EP plasmid introduction, it is preferable to use a Balan-CD-HEK293 medium.

In addition, in a case where the concentration of waste products and nutrients in the culture medium can be maintained at a certain quality by perfusion culture or the like in the preculture, the buffer exchange may be omitted, and the preculture suspension may be applied to the EP as it is. By omitting the concentration and culture medium exchange step, it is possible to achieve step simplification, reduction of contamination risk, improvement of process stabilization and reproducibility, and reduction of cost.

(3) Addition and mixing of nucleic acid

As the nucleic acid, a nucleic acid encoding a target virus is used, and preferably a exogenous recombinant nucleic acid encoding a virus is used.

The target virus means a virus that is produced by introducing an exogenous nucleic acid into a cell. Examples of the target virus include a non-enveloped virus. More specific examples thereof include an adeno-associated virus, an adenovirus, a lentivirus, a baculovirus, and a retrovirus, and among these, an adeno-associated virus is preferable.

The non-enveloped virus is known in the related art, and is described in WO2015/005430A, the contents of which are incorporated herein by reference.

Adeno-associated virus (AAV) refers to a small, incompletely replicative, non-enveloped virus containing single-stranded DNA having about 4,700 bases, from the family of Parvoviridae and Dependoparvovirus. It is known that there are more than 100 serum types in AAV, and that the host range and the characteristics of the virus differ depending on the difference in the serum types. Serum type 2 (AAV2) is one of the serum types that have been widely studied for a long time, and it is known that the host range is very wide. The serum type 1 (AAV1), the serum type 5 (AAV5), and the serum type 6 (AAV6) are serum types having higher tissue tropisms. It is said that AAV1 has high gene introduction efficiency into muscle, liver, airway, central nervous system, and the like, AAV5 has high gene introduction efficiency into central nervous system, liver, retina, and the like, and AAV6 has high gene introduction efficiency into heart, muscle, liver, and the like. In the present invention, the serum type 2 or the serum type 5 is preferably used. The serum type 5 is particularly preferable.

The adeno-associated virus gene refers to a gene composed of one or a plurality of nucleic acid sequences derived from the serum types of one or a plurality of adeno-associated viruses. The adeno-associated virus gene is preferably a gene involved in replication and packaging of AAV and a gene encoding an AAV constituent protein.

AAV is a non-enveloped virus that proliferates in the presence of helper viruses such as adenovirus and herpesvirus. In the preparation of AAV to be used for gene therapy or nucleic acid transfer, classically, AAV replication has been performed by co-infecting host cells with an adenovirus. In addition, a gene responsible for the helper action of adenovirus has been clarified, and a plasmid incorporating this gene has also been used. For example, a plasmid containing a Rep gene and a Cap gene, an adenovirus helper plasmid, and a plasmid containing a gene for treatment or prevention are simultaneously transfected into cells, and can thus be packaged as recombinant AAV (rAAV).

The Rep gene and the Cap gene encode proteins involved in the replication and packaging of the virion. In the wild type, the Rep gene is expressed from the p5 promoter and the p19 promoter. The Cap region expresses VP1, VP2, and VP3. Examples of the promoter that is naturally carried by the Cap gene can include a p40 promoter.

The adeno-associated virus gene (such as a Rep gene and a Cap gene) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base are made to the wild-type adeno-associated virus gene (such as a Rep gene and a Cap gene), the number of the modified bases is preferably 1 to 20, more preferably 1 to 10, and still more preferably 1 to 3. The modified base sequence of the adeno-associated virus gene exhibits preferably 85% or more sequence identity, more preferably 90% or more sequence identity, still more preferably 95% or more sequence identity, and even still more preferably 98% or more sequence identity with the base sequence of the wild-type adeno-associated virus gene.

In a case where the Rep gene and the Cap gene are introduced into a cell, a vector containing the Rep gene and the Cap gene can be introduced into the cell. The arrangement of the Rep gene and the Cap gene in a vector is not particularly limited, the Rep gene may be located upstream of the Cap gene or downstream of the Cap gene, and the Rep gene is located preferably upstream of the Cap gene.

The Rep gene means a region of the AAV genome encoding a virus replication protein that is known to those skilled in the art and that is collectively required for replication of a virus genome, or its functional homolog, for example, a human herpesvirus 6 (HHV-6) Rep gene, or the like (mediating AAV-2 DNA replication is known). Accordingly, the coding region of the Rep gene includes at least a gene encoding REP78 and REP68 (long form of REP protein) and REP52 and REP40 (short form of REP protein) of AAV, or a functional homologue thereof. The coding region of the Rep gene used in the present invention may be derived from any AAV serum type, but is preferably derived from AAV2. Examples of those derived from AAV2 include REP78 and REP68, and REP52 and REP40, and ITR.

The Cap gene means a region in the AAV genome encoding a capsid protein of a virus known to those skilled in the art. Examples of these capsid proteins are AAV capsid proteins VP1, VP2 and VP3. The Cap gene used in the present invention may be derived from any AAV serum type, but is preferably derived from AAV2 or AAV5. AAV2 is particularly preferable.

As the vector containing the Rep gene and the Cap gene, for example, a plasmid, a nucleic acid sequence derived from a virus, or an artificially designed nucleic acid can be used, and a plasmid is preferable.

In the present invention, a gene for treatment or prevention may be introduced into cells. It is preferable that the gene for treatment or prevention is transferred in a state of being sandwiched between ITRs.

As the gene for treatment or prevention, a gene that is incomplete or lost in the genome of the target cell, a gene encoding non-natural protein having a desired biological or therapeutic effect (for example, antiviral function), or the like can be used, but the gene is not particularly limited. Specific examples of the gene for treatment or prevention include a gene used for treatment or prevention of infectious diseases, inflammatory diseases, autoimmunity, chronic and contagious diseases (including disorders such as acquired immune deficiency syndrome (AIDS), cancer, nervous system diseases, cardiovascular diseases, and hypercholesterolemia), various blood diseases such as anemia and hemophilia, or gene deficiency (for example, cystic fibrosis, Gaucher's disease, adenosine deaminase (ADA) deficiency, emphysema, and the like).

The gene for treatment or prevention may be several antisense oligonucleotides (for example, a short-chain oligonucleotide complementary to a sequence around a translation initiation site (AUG codon) of mRNA) that are useful in antisense therapy against cancer and viral disease.

The gene for therapy or prophylaxis may be linked to a promoter for expressing the gene for treatment or prevention. The promoter for expressing a gene for treatment or prevention is not particularly limited, but examples thereof can include a cytomegalovirus-derived promoter (including an enhancer as desired), an SV40 early promoter, a human elongation factor-1$\alpha$ (EF-1$\alpha$) promoter, a human ubiquitin C promoter, a retrovirus Rous sarcoma virus LTR promoter, a dihydrofolate reductase promoter, a $\beta$-actin promoter, a phosphoglycerate kinase (PGK) promoter, and the like. The gene for treatment or prevention and the promoter for expressing the gene are preferably flanked by the ITR sequences.

As the vector containing a gene for treatment or prevention, for example, a plasmid, a virus-derived sequence, an artificially designed nucleic acid, or the like can be used, and a plasmid is preferable.

In the present invention, it is preferable that a virus helper gene derived from an adenovirus is transferred into cells. The virus helper gene is a non-adeno-associated virus gene for enabling replication and packaging of an adeno-associated virus. As the virus helper gene, a gene derived from a virus of another species other than the adeno-associated virus is used. Specific examples of the virus helper gene include a virus helper gene derived from an adenovirus or a herpesvirus, and the virus helper gene is preferably derived from an adenovirus.

Examples of the virus helper gene derived from adenovirus can include E1A, E1B, E2A, E4, and VA-RNA. In the host cell having all or a part of the E1 region, the region of the adenovirus genome necessary for replicating the AAV genome and packaging of the AAV genome into the capsid to form a AAVvirion is the E2A region, the E4 region, and the VA-RNA region. For the function by the E4 region, the 34 kDa E4 protein encoded by the open reading frame 6 (E4ORF6) of the E4 region is required for replicating the AAV. Preferably, the virus helper gene is an E2 gene, an E4 gene, or a VA-RNA gene. The VA-RNA gene is preferably a VA-RNAI gene.

The adenovirus-derived virus helper gene (such as an E1A, an E1B, an E2A, an E4, and a VA-RNA) may be a wild-type gene, but a gene in which modifications such as substitution, deletion, insertion, or addition of a base have been made to the wild-type gene may be used as long as it exhibits an inherent function.

In a case where modifications such as substitution, deletion, insertion, or addition of a base are made to the wild-type virus helper gene, the number of the modified bases is preferably 1 to 20, more preferably 1 to 10, and still more preferably 1 to 3. The modified base sequence of the virus helper gene exhibits preferably 85% or more sequence identity, more preferably 90% or more sequence identity, still more preferably 95% or more sequence identity, and even still more preferably 98% or more sequence identity with the base sequence of the wild-type virus helper gene.

In a case of introducing a virus helper gene into a cell, a vector containing the virus helper gene can be introduced into the cell.

As the vector containing a virus helper gene, for example, a plasmid, a virus-derived sequence, an artificially designed nucleic acid, or the like can be used, and a plasmid is preferable.

The virus helper gene is preferably under the control of a promoter and may be under the control of a promoter capable of regulating expression.

The specific examples of the promoter are not particularly limited, but can include a cytomegalovirus-derived promoter (CMV promoter) (including an enhancer as desired), an SV40 early promoter, a human elongation factor-1$\alpha$ (EF-1$\alpha$) promoter, a human ubiquitin C promoter, a retrovirus Rous sarcoma virus (RSV) LTR promoter, a dihydrofolate reductase promoter, a $\beta$-actin promoter, and a phosphoglycerate kinase (PGK) promoter.

As described above, the nucleic acid preferably contains one or more of an adeno-associated virus gene and a virus helper gene, and the nucleic acid preferably contains at least one or more virus helper genes. Still more preferably, the nucleic acid contains four or more genes selected from the group consisting of a gene for treatment or prevention, a Rep gene, a Cap gene, an E2 gene, an E4 gene, and a VA-RNA1 gene.

As described above, in the present invention, the nucleic acid can be introduced into the cell using a plurality of plasmids. Provided that the form of the introduced nucleic acid is not limited as long as the purpose of introducing the nucleic acid encoding the gene necessary for virus production into the cell is achieved. For example, a large plasmid in which the above-described gene is carried in one plasmid may be used. Provided that since in electroporation, as the size of the plasmid increases, the cell damage during electroporation tends to increase, the size of the plasmid is preferably 15 kbp or less.

The method for manufacturing a virus may be any of a triple transfection (TT) method or a packaging cell method.

The TT method is a method in which a plasmid containing a Rep gene and a Cap gene, an adenovirus helper plasmid, and a plasmid containing a transgene (for example, a gene for desired therapy or prophylaxis) are simultaneously transfected into cells, and can thus be packaged as recombinant AAV (rAAV).

The packaging cell method is a method of producing AAV by introducing, to the cell in which a part of the gene in the TT method is incorporated into a chromosome in advance, the remaining genes into a cell by transfection.

To obtain a desired cell concentration and a desired nucleic acid concentration, the liquid volume and cell concentration of the cell suspension before nucleic acid mixing, and the liquid volume and nucleic acid concentration of the nucleic acid stock solution can be appropriately adjusted and changed. The method of adding and mixing is not particularly limited, as long as shear damage to the cells is suppressed to a minimum and uniform dispersion of the cells and the nucleic acid is achieved. In a case of treating a small amount, the nucleic acids may be mixed by pipetting and directly added and mixed in the electroporation device. In a case of treating a large amount, from the viewpoint of reducing the contamination risk, stirring and mixing in a sterile connected dedicated container or continuous flow mixing is preferable.

(4) Electroporation (gene introduction)

The type of power supply does not matter as long as the required conditions of the applied voltage (required electric field E $\times$ gap) and the power capacity (pulse current, pulse duration, pulse interval) are satisfied. The function generator is several tens of V and several tens of $\mu$m, and the function generator + power amplifier is several hundreds of V and 2 mm gap. The dedicated pulse power supply (DC power supply + capacitor + power switch circuit) is ~ several kV and ~ 1 cm gap.

The liquid feeding pump and the liquid feeding method may be any of a syringe pump, a tube pump, a magnet pump, a diaphragm pump, or the like, or may be pressurized air feeding.

The electroporation may be any of batch electroporation (single-use cuvette (several tens of $\mu$L to about 1 mL, pipetting work), continuous batch electroporation (several mL to several hundreds of mL), or flow electroporation (several mL to several tens of L). In a case where the treatment amount (amount of culture solution) is large, flow electroporation is preferable. In the case of flow electroporation, a liquid feeding method with reduced pulsation is preferable.

The device for performing electroporation is not particularly limited. The device is known in the related art, and is described in WO2022/224803A, WO2023/157673A, and WO2023/223931A, the contents of which are incorporated herein by reference.

As an example of a device for performing flow electroporation, the electroporation device shown in FIGS. 1 and 2 can be used. FIG. 1 is a top view of the electroporation device 100, and FIG. 2 is a cross-sectional view of the electroporation device 100.

In the electroporation device 100, the upper electrode 41a held by the upper electrode holding plate 10 and the lower electrode 41b held by the lower electrode holding plate 20 are installed to face each other to constitute an electrode pair 41. A power supply (not shown) is connected to the upper electrode 41a and the lower electrode 41b. D represents an inter-electrode distance (gap). In FIG. 1, L represents an electrode length, and W represents an electrode width (flow channel width). In FIG. 2, a1 represents a front surface portion of the upper electrode, a2 represents a rear surface portion of the upper electrode, b1 represents a front surface portion of the lower electrode, and b2 represents a rear surface portion of the lower electrode. A flow channel plate 30 is provided between the upper electrode holding plate 10 and the lower electrode holding plate 20. The fluid introduced from the inflow port 50 is discharged from the outflow port 52 through the opening portion (flow channel) 32.

It is preferable that the supply reservoir, the liquid feeding tube, the electroporation device, and the collection reservoir are aseptically connected to each other. That is, in the present invention, it is preferable that the liquid feeding is performed aseptically in the nucleic acid introduction step and the culture step. The preculture step (including concentration, culture medium exchange, and mixing system) and the subsequent culture step may be continuously connected or may be separated.

In the present invention, the introduction of the nucleic acid is preferably performed only via electroporation. In the present invention, the nucleic acid is preferably introduced into the cells in the absence of the transfection reagent. In the present invention, it is preferable that additional nucleic acid introduction is not performed after the nucleic acid introduction step via electroporation.

In the present invention, the CNET product represented by the following expression in electroporation is $1 \times 10^4$ or more and $1 \times 10^7$ or less.

$$\sum_{k=1}^{n} C|N_k E_k T_k| \quad \text{or} \quad \sum_{k=1}^{n} C|N_k E_k T_k| + \sum_{l=1}^{m} C|N_l E_l T_l|$$

In the formula, C represents a nucleic acid concentration in terms of μg/mL,

$N_k$ represents the number of pulses, and $N_k$ is an integer of 1 or more,
$E_k$ represents a pulsed electric field in terms of V/cm, and $E_k$ is 500 V/cm or more and 2,000 V/cm or less,
$T_k$ represents a pulse duration in terms of ms,
$N_l$ represents the number of pulses, and $N_l$ is an integer of 1 or more,
$E_l$ represents a pulsed electric field in terms of V/cm, and $E_l$ is 50 V/cm or more and less than 500 V/cm,
$T_l$ represents a pulse duration in terms of ms,
k represents an integer from 1 to n,
l represents an integer from 1 to m,
n represents an integer of 1 or more, and
m represents an integer of 1 or more.

C (nucleic acid concentration in terms of μg/mL) is preferably 10 to 500 μg/mL, more preferably 30 to 450 μg/mL, still more preferably 50 to 400 μg/mL, and particularly preferably 60 to 375 μg/mL.

In addition, in a case of $\sum_{k=1}^{n} C|N_k E_k T_k|$, the nucleic acid concentration is preferably 100 to 300 μg/mL, and in a case of $\sum_{k=1}^{n} C|N_k E_k T_k| + \sum_{l=1}^{m} C|N_l E_l T_l|$, the nucleic acid concentration is preferably 10 to 100 μg/mL.

Since the mixed suspension of the cells and the nucleic acid is produced by adding (mixing) the plasmid stock solution to the cell suspension, the plasmid stock solution concentration is inevitably diluted to a certain extent. The upper limit of the concentration of the plasmid stock solution is about several mg/mL (more than the upper limit cannot be dissolved and precipitated or aggregated), and it is difficult to adjust a mixed solution having a plasmid concentration of 500 μg/mL or more. In addition, in a case where the nucleic acid concentration C is low, to secure a desired CNET product (to obtain the required amount of plasmid to be introduced into the cells), it is necessary to increase the NET to further increase the electrophoresis distance. There is a trade-off in which efficiency decreases because of an increase in cell damage or an increase in NT.

$N_k$ represents the number of pulses, and $N_k$ represents an integer of 1 or more, preferably represents an integer of 1 or more and 10 or less, more preferably represents an integer of 1 or more and 5 or less, and still more preferably represents an integer of 1 or more and 3 or less.

$N_l$ represents the number of pulses, and Ni represents an integer of 1 or more, preferably represents an integer of 1 or more and 10 or less, more preferably represents an integer of 1 or more and 5 or less, and still more preferably represents an integer of 1 or more and 3 or less.

$N_k$+$N_l$ preferably represents an integer of 1 or more and 10 or less, and more preferably represents an integer of 1 or more and 5 or less.

In a case where a plurality of pulses are performed, the interval is preferably 500 ms or less.

n represents an integer of 1 or more, preferably represents an integer of 1 or more and 10 or less, more preferably represents an integer of 1 or more and 5 or less, and still more preferably represents an integer of 1 or more and 3 or less.

m represents an integer of 1 or more, preferably represents an integer of 1 or more and 10 or less, more preferably represents an integer of 1 or more and 5 or less, and still more preferably represents an integer of 1 or more and 3 or less.

$E_k$ represents a pulsed electric field in terms of V/cm, and $E_k$ is 500 V/cm or more and 2,000 V/cm or less. By setting $E_k$ to 2,000 V/cm or less, it is possible to prevent cell damage and a decrease in introduction efficiency due to heat generation, boiling, and discharge. By setting $E_k$ to 500 V/cm or more, a sufficient cell membrane potential can be secured, and perforation can be caused.

$T_k$ represents a pulse duration in terms of ms, and is preferably 0.01 ms to 100 ms, more preferably 0.05 ms to 20 ms, and still more preferably 0.1 ms to 10 ms. In a case where the pulse duration is insufficient, sufficient perforation is not formed in the cell membrane, which causes a decrease in gene introduction efficiency. On the other hand, in a case where the pulse duration is too long, excessive perforation of cell membranes, or boiling or discharge due to heat generation of the suspension occurs, which causes a decrease in gene introduction efficiency or a decrease in cell viability.

$E_l$ represents a pulsed electric field in terms of V/cm, and $E_l$ is 50 V/cm or more and less than 500 V/cm. By setting $E_l$ to less than 500 V/cm, it is possible to prevent perforation action (damage). By setting $E_l$ to 50 V/cm or more, a decrease in electrophoresis rate can be prevented, the required time can be shortened, and a decrease in the electrophoresis efficiency and introduction efficiency can be prevented.

$T_l$ represents a pulse duration in terms of ms, and is preferably 0.01 ms to 1 s, more preferably 0.1 ms to 200 ms, and still more preferably 1 ms to 100 ms. In the low-electric field electrophoresis pulse, since cell damage and heat generation are suppressed, it is possible to apply a pulse longer than the perforation pulse. Provided that the application of the excessive long pulse causes a decrease in the gene introduction efficiency into the perforation formation site on the cell membrane due to the sedimentation or rotation of cells during the pulse application, or causes a decrease in the cell viability caused by the elution of the metal or the generation of radicals due to the promotion of the electrolysis reaction at the electrode.

In the present invention, the CNET product is $1.0 \times 10^4$ or more and $1.0 \times 10^7$ or less, preferably $1.0 \times 10^5$ or more and $1.0 \times 10^6$ or less, more preferably $2.0 \times 10^5$ or more and $1.0 \times 10^6$ or less, still more preferably $2.0 \times 10^5$ or more and $5.0 \times 10^5$ or less, and particularly preferably $2.0 \times 10^5$ or more and $4.0 \times 10^5$ or less.

A virus cannot be produced in a case where the necessary nucleic acid is not introduced into the cell, but in a case where an excessive nucleic acid is introduced under an excessive electroporation condition, the cell activity is decreased due to perforation damage, and the cell viability is decreased, whereby the titer is decreased. In addition, the toxicity at the time of REP expression decreases the cell activity, and the cell viability decreases, thereby decreasing the titer. In addition, even under the conditions suitable for virus production (high titer conditions), to further increase the Full rate (the ratio of the amount of the capsid containing the full-length gene to the total amount of the capsid), it is preferable to cell-level and nuclear-level reaction control by more precise control of the amount of the introduced plasmid to avoid (suppress) excessive capsid production (protein production) and to maintain the replication (nucleic acid replication) of the gene for treatment or prevention and the packaging reaction rate into the capsid.

In the present invention, it is preferable that the CNET product is represented by

$$\sum_{k=1}^{n} C|N_k E_k T_k| + \sum_{l=1}^{m} C|N_l E_l T_l|$$

, and

$$\sum_{k=1}^{n} C|N_k E_k T_k| < \sum_{l=1}^{m} C|N_l E_l T_l|$$

is satisfied.

FIGS. 3 to 6 shows an example of a pulse waveform of electroporation. In FIGS. 3 to 6, the vertically long rectangle indicates a perforation pulse, and the laterally long rectangle indicates an electrophoresis pulse.

The upper part of FIG. 3 shows that the electroporation is performed in the order of the perforation pulse, the perforation pulse, and the electrophoresis pulse. The middle part of FIG. 3 shows that the electroporation is performed in the order of the perforation pulse, the electrophoresis pulse, and the perforation pulse. The lower part of FIG. 3 shows a case where the perforation pulse and the electrophoresis pulse are integrated.

FIG. 4 shows that the polarity of the pulse may be both positive and negative.

FIG. 5 shows that the pulse waveform may be a damped waveform. That is, the intensity of the pulse may be damped between a single perforation pulse and a single electrophoresis pulse. In a case where the damped pulse is used, the pulsed electric field ($E_k$ and $E_l$) can be a time-averaged value or a peak voltage value. The ET value is a time integral of the voltage waveform pulse.

FIG. 6 shows an example of a pulse waveform.

(5) Culture for virus production

In the present invention, the virus is produced by performing a culture step of culturing cells into which a nucleic acid has been introduced.

The culture method of the culture step is not particularly limited, and may be batch culture, fed-batch culture, or the like, or may be any of shaking culture, stirring culture, standing culture, or adhesion culture.

The culture temperature of the cells is not particularly limited, and the culture of the cells is performed at a temperature at which the cells can survive. The culture temperature is generally 25°C to 45°C, preferably 30°C to 42°C, more preferably 35°C to 40°C, and 37°C as an example. The $CO_2$ concentration is generally 3 to 10% $CO_2$, preferably 5 to 10% $CO_2$, and 8% $CO_2$ as an example.

The pH of the culture solution is preferably 6.0 to 8.0, more preferably 6.5 to 7.5, and still more preferably 6.6 to 7.0. In addition, the $CO_2$ of the culture solution is preferably 200 mmHg or less and more preferably 160 mmHg or less.

As the culture conditions, the suitable conditions in the preculture may be applied as they are. In addition, since there are effects of perforation damage due to electroporation, expression of introduced genes, immune response due to virus accumulation in cells, toxicity, and the like, the culture conditions may be optimized to have lower damage than the preculture (proliferation culture).

The cell density is preferably equal to or less than that in the preculture. The cell density is preferably $1 \times 10^6$ to $10 \times 10^6$ cells/mL in a case of the batch culture, and preferably $1 \times 10^6$ to $20 \times 10^6$ cells/mL in a case of the fed-batch culture.

The cell concentration may be diluted at the time of the main culture step after the electroporation.

It is preferable that the shearing is equal to or less than that of the preculture, and it is preferable that the oxygen concentration is equal to or higher than that of the preculture. The nutritional components are the same as those in the preculture.

The period of the virus production step is preferably 24 hours or more and 90 days or less, more preferably 24 hours or more and 30 days or less, still more preferably 24 hours or more and 20 days or less, even still more preferably 24 hours or more and 10 days or less, yet even still more preferably 24 hours or more and 7 days or less, and particularly preferably 24 hours or more and 72 hours or less. Since electroporation does not have an intracellular introduction, endosome escape, and a PEI slow release process in principle unlike the PEI method, the nuclear reaching to expression speed of the introduced gene is faster than that of the PEI method. In the typical PEI method, the virus production period is 72 hours, whereas in the EP, it is preferably about 48 hours.

As the culture container, a flask or a bioreactor can be used, but it is not particularly limited. The shape of the culture container is not particularly limited. The size of the culture container is a size in which culture can be performed in a culture scale described later.

The culture scale is not particularly limited, and the cells can be cultured in a culture medium of any volume, for example, the cells can be cultured in a culture medium of 1 mL to 3,000 L, preferably 1 L to 2,500 L, more preferably 10 L to 1,000 L, and particularly preferably 50 L to 500 L.

In cell culture, a gas containing oxygen can be introduced into a culture solution using a sparger. It is preferable that the dissolved oxygen concentration of the culture solution is adjusted by introducing a gas containing oxygen into the culture solution. The dissolved oxygen concentration in the culture solution can be appropriately set and is not particularly limited; however, it is 10% to 150%, preferably 15% to 120%, and more preferably 20% to 100% in a case where the saturated dissolved oxygen concentration in the liquid at 37°C in air at 1 atm is set to 100%.

The pore diameter of the sparger is not particularly limited, but is preferably 5 $\mu$m to 100 $\mu$m, and more preferably 10 $\mu$m to 50 $\mu$m.

The ventilation amount of the gas containing oxygen is not particularly limited, but is generally 0.001 to 1.0 vvm and preferably 0.005 to 0.5 vvm. VVM is volume per volume per minute.

The culture may be performed while stirring by shaking. The stirring speed in the case of stirring by shaking is generally 50 rpm to 200 rpm and preferably 80 to 180 rpm. The stirring culture may be rotary stirring culture using an impeller, a propeller, a paddle, or the like in a reactor. For example, a three-blade propeller, a two-blade paddle, or the like can be used. The size of the impeller, the propeller, or the paddle is set according to the size of the culture tank.

The stirring speed in the case of stirring by rotating is generally 50 rpm to 400 rpm, and preferably 80 to 350 rpm. In addition, stirring may be performed by wave-type shaking stirring or vertical movement of the stirring blade, but the stirring is not particularly limited.

In the culture, a membrane separation treatment step of passing a cell suspension extracted from a culture tank through a separation membrane to separate the cell suspension into a cell-containing liquid and a permeated liquid may be performed. In this operation, the cell suspension extracted from the culture tank is separated into a cell-containing liquid having a cell concentration higher than that of the cell suspension and a permeated liquid having a cell concentration lower than that of the cell suspension.

The membrane separation treatment step is preferably tangential filtration, more preferably alternating tangential flow (ATF) or tangential flow, and most preferably ATF. Examples of the filter that can perform ATF include SuATF10-S02PES, F2 RF02PES, or the like, manufactured by Repligen Corporation.

Examples of the material of the membrane used in the membrane separation treatment step include polyethersulfone, modified polyethersulfone, mixed cellulose ester, and the like.

The pore diameter of the membrane used in the membrane separation treatment step is preferably 0.1 μm to 0.4 μm and more preferably 0.15 μm to 0.3 μm.

In the present invention, it is preferable that the cells are in a suspension in the nucleic acid introduction step and the culture step, and the suspension is a culture medium.

In the culture according to the embodiment of the present invention, which includes the preculture, cell bleeding may be performed by withdrawing a part of the culture solution. By performing the cell bleeding, the cell density can be maintained at a predetermined value.

The cell bleeding can be performed by the following method, as an example. After measuring the cell concentration, in a case of withdrawing the culture solution, the pump is operated at a rate of 0.3 vvd to 3 vvd. The cell liquid obtained by cell bleeding may be transferred to a culture tank separate from the preculture. The cell bleeding is performed once or more a day, and the cell bleeding performed last in a day is preferably performed within 5 hours and more preferably within 3 hours from the cell bleeding performed first. After the cell bleeding is completed in a day, an amount of cell culture medium equal to the amount of cell suspension reduced by the cell bleeding is added, thereby returning the total culture volume in the preculture to the level before the cell bleeding, and the culture is continued.

The cell bleeding can also be performed by automatic control. In a case where the cell bleeding was automatically controlled, the cell bleeding can be continuously performed automatically, for example, using a FUTURA sensor manufactured by ABER Instruments Ltd. while monitoring the electrostatic capacity of the culture solution. As an example, the method can be performed by the following method.

(1) An electrostatic capacity serving as a target is set.
(2) A weight control value of the culture tank is set.
(3) The electrostatic capacity of the culture solution is measured at a period of 0.1 s or less.
(4) A pump for withdrawing the culture solution is driven at a rate of 0.3 vvd to 3 vvd in a case where the electrostatic capacity of the culture solution exceeds the target.
(5) A culture medium is automatically supplied into the culture tank while the weight of the culture tank is measured, to keep the liquid volume of the culture solution approximately constant.
(6) The pump for withdrawing the culture solution is stopped in a case where the electrostatic capacity of the culture solution is smaller than the target by 0.01% or more.

The cell bleeding rate is preferably 10% to 50%/day and more preferably 20% to 50%/day. The cell bleeding rate is a proportion of the culture solution withdrawn per day with respect to the total amount of the culture solution.

(6) Recovery and purification of virus

The method according to the embodiment of the present invention preferably includes recovering the produced virus.

The virus particles produced in the cell are present in the culture solution or in the cell (in the nucleus). The extraction from the inside of the cell (destruction/dissolution of the cell membrane and the nuclear membrane) may be performed by a freeze-thawing method, or may be performed by adding a surfactant (such as Triton X) and stirring. The separation of the virus and the cell debris can be performed by centrifugation or depth filtration.

The virus may be purified by performing TFF (concentration/buffer exchange), affinity chromatography, or AEX (anion exchange chromatography) treatment as necessary. In this manner, it is possible to remove cell debris, cell-derived nucleic acids, and cell-derived proteins. Alternatively, a commercially available virus purification kit (such as an AAV purification kit) may be used. For example, an AAVpro (registered trademark) Purification Kit Maxi/Midi manufactured by Takara Bio Inc. or the like can be used.

(7) Analysis

The titer of the virus produced by the method according to the embodiment of the present invention can be measured by a normal method known to those skilled in the art. For example, the cell culture solution after the culture is collected, the cells are disrupted by freeze-thaw, and then the supernatant is collected by centrifugation. The gDNA (genomic DNA) and the residual plasmid can be digested by adding $MgCl_2$ and Benzonase to the collected supernatant to react. It is possible to measure the virus titer (vg/mL) by using the sample containing the target virus obtained as described above as a droplet digital PCR (ddPCR) sample, and performing ddPCR to measure the number of copies of intra-viral genome.

Furthermore, the Full ratio can be calculated from the ratio of the capsid particle titer (Vp/mL) measured using the ELISA kit to the genome titer (vg/mL) calculated by ddPCR measurement. Alternatively, the Full/Empty ratio may be obtained by analyzing the sample solution after virus extraction and purification by HPLC measurement, isoelectric focusing, or the like in the same measurement system.

In the present invention, in the virus produced in the culture step, the ratio (Full ratio) of the amount of the capsid containing the full-length gene to the total amount of the capsid is preferably 5% or more, more preferably 10% or more, still more preferably 20% or more, and particularly preferably 30% or more.

FIG. 9 is a schematic view showing a continuous production apparatus for AAV in which a perfusion culture device are connected to a flow electroporation device. HEK293 cells are precultured in a perfusion culture device having ATF to a high cell concentration (for example, $40 \times 10^6$ cells/ml). In the following table, M indicates $\times 10^6$. Once a day, an amount corresponding to a fixed percentage of cell-containing solution is withdrawn from the perfusion culture device, and a gene introduction is continuously performed using a flow electroporation device aseptically connected, by mixing the solution with a plasmid. Although the concentration device is not shown, the cells may be concentrated to a cell concentration of $80 \times 10^6$ cells/ml or more using ATF. In the perfusion culture device after the withdrawing, the same amount of a culture medium as the withdrawn amount is added, the temporarily decreased cell concentration is restored to the same cell concentration as the previous day by culturing for 24 hours, and the gene introduction is performed by withdrawing the cell-containing solution in the same manner on the next day and thereafter. By repeating this cycle using the perfusion culture device, a large amount of high-concentration cells can be supplied. In the cell-containing solution after gene introduction, the oxygen concentration is higher than that in the preculture.

A cell-containing solution into which a gene (plasmid) has been introduced by a flow electroporation method is supplied to the downstream main culture device, and AAV production culture is performed for 48 hours. The main culture device and the control device are operated by switching between days. The devices may be prepared for the number of production days, or a certain number of devices may be repeatedly used. In the case of repeated operation, a device in which the AAV production culture for 48 hours is completed may be reused after washing and sterilization, or a new single-use reactor may be installed in the main culture device. Such an operation makes it possible to achieve the continuous production of AAV. In the PEI method, which requires control the complex formation between PEI and a plasmid and the introduction reaction of the complex into cells, there is an issue that the gene introduction efficiency under a high cell concentration condition decreases, and in the batch AAV manufacture by the existing PEI method, it is common to operate at a cell concentration of about $2 \times 10^6$ to $4 \times 10^6$ cells/ml. On the other hand, the electroporation method has an advantage that gene introduction and expression can be performed with high efficiency at a high cell concentration.

In a case where cells having a concentration of $40 \times 10^6$ cells/ml are continuously supplied at 40% of the device volume per day for 25 days by perfusion culture, and gene introduction by a flow electroporation method and AAV production culture are performed on all cells, the cell concentration is 10 to 20 times, the supply amount of the cell suspension is 0.4 times/day $\times$ 25 days, and a total of 100 to 200 times of gene introduction treatment to cells is possible. Therefore, mass production of AAV is possible even in a small-scale culture device.

The present invention will be more specifically described using the following examples; however, it is not limited by the examples.

## Examples

### (1) Preculture

As cells, a suspension HEK293 cell line Viral Production Cells 2.0 (VPCs 2.0) manufactured by Thermo Fisher Scientific, Inc. was used. As a culture medium, a Balan-CD-HEK293 medium manufactured by FISI was used. As the culture device, an animal cell culture device BCP1L reactor manufactured by Biott Co., Ltd. was used. Perfusion culture was performed at a cell density of $40 \times 10^6$ cells/mL, 40% cell bleeding, and 37°C $\pm$ 1.5°C. FIG. 7 shows a schematic view of perfusion culture.

### (2) Concentration and culture medium exchange

In the batch electroporation (BEP) experiment, a cell suspension of $40 \times 10^6$ cells/mL withdrawn from a perfusion culture reactor was centrifuged at 200 x g for 5 minutes, the supernatant was removed, and then the cells were resuspended in fresh Balan-CD-HEK293 medium to adjust the cell concentration to 120 Mcells/mL.

In the flow electroporation (FEP) experiment, a cell suspension of $40 \times 10^6$ cells/mL withdrawn from the perfusion culture reactor was used as it was.

(3) Plasmid

A plasmid for AAV production was used.

pAAV-GFP (SEQ ID NO: 1)
pAAV-RC5 (SEQ ID NO: 2)
pHelper (SEQ ID NO: 3)

FIG. 8 shows schematic diagrams of pAAV-GFP, pAAV-RC5, and pHelper.

(SEQ ID NO: 1)

gatccacggg tggcatccct gtgacccctc cccagtgcct ctcctggccc tggaagttgc 60
cactccagtg cccaccagcc ttgtcctaat aaaattaagt tgcatcattt tgtctgacta 120
ggtgtccttc tataatatta tggggtggag gggggtggta tggagcaagg ggcaagttgg 180
gaagacaacc tgtagggcct gcggggtcta ttgggaacca agctggagtg cagtggcaca 240
atcttggctc actgcaatct ccgcctcctg ggttcaagcg attctcctgc ctcagcctcc 300
cgagttgttg ggattccagg catgcatgac caggctcagc taatttttgt ttttttggta 360
gagacggggt ttcaccatat tggccaggct ggtctccaac tcctaatctc aggtgatcta 420
cccaccttgg cctcccaaat tgctgggatt acaggcgtga accactgctc ccttccctgt 480
ccttatcgat agatctagga acccctagtg atggagttgg ccactccctc tctgcgcgct 540
cgctcgctca ctgaggccgg gcgaccaaag gtcgcccgac gcccgggctt tgcccgggcg 600
gcctcagtga gcgagcgagc gcgcagctgc ctgcaggcag cttggcactg gccgtcgttt 660
tacaacgtcg tgactgggaa aaccctggcg ttacccaact taatcgcctt gcagcacatc 720
ccccttcgc cagctggcgt aatagcgaag aggcccgcac cgatcgccct tcccaacagt 780
tgcgcagcct gaatggcgaa tggcgcctga tgcggtattt tctccttacg catctgtgcg 840
gtatttcaca ccgcatacgt caaagcaacc atagtacgcg ccctgtagcg gcgcattaag 900
cgcggcgggt gtggtggtta cgcgcagcgt gaccgctaca cttgccagcg ccctagcgcc 960
cgctcctttc gctttcttcc cttcctttct cgccacgttc gccggctttc cccgtcaagc 1020
tctaaatcgg gggctccctt tagggttccg atttagtgct ttacggcacc tcgaccccaa 1080
aaaacttgat ttgggtgatg gttcacgtag tgggccatcg ccctgataga cggtttttcg 1140
ccctttgacg ttggagtcca cgttctttaa tagtggactc ttgttccaaa ctggaacaac 1200
actcaaccct atctcgggct attcttttga tttataaggg attttgccga tttcggccta 1260
ttggttaaaa aatgagctga tttaacaaaa atttaacgcg aattttaaca aaatattaac 1320
gtttacaatt ttatggtgca ctctcagtac aatctgctct gatgccgcat agttaagcca 1380
gccccgacac ccgccaacac ccgctgacgc gccctgacgg gcttgtctgc tcccggcatc 1440
cgcttacaga caagctgtga ccgtctccgg gagctgcatg tgtcagaggt tttcaccgtc 1500
atcaccgaaa cgcgcgagac gaaagggcct cgtgatacgc ctatttttat aggttaatgt 1560
catgataata atggtttctt agacgtcagg tggcactttt cggggaaatg tgcgcggaac 1620
ccctatttgt ttatttttct aaatacattc aaatatgtat ccgctcatga gacaataacc 1680
ctgataaatg cttcaataat attgaaaaag gaagagtatg agtattcaac atttccgtgt 1740

cgcccttatt cccttttttg cggcattttg ccttcctgtt tttgctcacc cagaaacgct 1800
ggtgaaagta aaagatgctg aagatcagtt gggtgcacga gtgggttaca tcgaactgga 1860
tctcaacagc ggtaagatcc ttgagagttt tcgccccgaa gaacgttttc caatgatgag 1920
cacttttaaa gttctgctat gtggcgcggt attatcccgt attgacgccg ggcaagagca 1980
actcggtcgc cgcatacact attctcagaa tgacttggtt gagtactcac cagtcacaga 2040
aaagcatctt acggatggca tgacagtaag agaattatgc agtgctgcca taaccatgag 2100
tgataacact gcggccaact tacttctgac aacgatcgga ggaccgaagg agctaaccgc 2160
ttttttgcac aacatggggg atcatgtaac tcgccttgat cgttgggaac cggagctgaa 2220
tgaagccata ccaaacgacg agcgtgacac cacgatgcct gtagcaatgg caacaacgtt 2280
gcgcaaacta ttaactggcg aactacttac tctagcttcc cggcaacaat taatagactg 2340
gatggaggcg gataaagttg caggaccact tctgcgctcg gcccttccgg ctggctggtt 2400
tattgctgat aaatctggag ccggtgagcg tgggtctcgc ggtatcattg cagcactggg 2460
gccagatggt aagccctccc gtatcgtagt tatctacacg acggggagtc aggcaactat 2520
ggatgaacga aatagacaga tcgctgagat aggtgcctca ctgattaagc attggtaact 2580
gtcagaccaa gtttactcat atatacttta gattgattta aaacttcatt tttaatttaa 2640
aaggatctag gtgaagatcc tttttgataa tctcatgacc aaaatccctt aacgtgagtt 2700
ttcgttccac tgagcgtcag accccgtaga aaagatcaaa ggatcttctt gagatccttt 2760
ttttctgcgc gtaatctgct gcttgcaaac aaaaaaacca ccgctaccag cggtggtttg 2820
tttgccggat caagagctac caactctttt tccgaaggta actggcttca gcagagcgca 2880
gataccaaat actgttcttc tagtgtagcc gtagttaggc caccacttca agaactctgt 2940
agcaccgcct acatacctcg ctctgctaat cctgttacca gtggctgctg ccagtggcga 3000
taagtcgtgt cttaccgggt tggactcaag acgatagtta ccggataagg cgcagcggtc 3060
gggctgaacg gggggttcgt gcacacagcc cagcttggag cgaacgacct acaccgaact 3120
gagataccta cagcgtgagc tatgagaaag cgccacgctt cccgaaggga gaaaggcgga 3180
caggtatccg gtaagcggca gggtcggaac aggagagcgc acgagggagc ttccaggggg 3240
aaacgcctgg tatctttata gtcctgtcgg gtttcgccac ctctgacttg agcgtcgatt 3300
tttgtgatgc tcgtcagggg ggcggagcct atggaaaaac gccagcaacg cggccttttt 3360
acggttcctg gccttttgct ggccttttgc tcacatgttc tttcctgcgt tatcccctga 3420
ttctgtggat aaccgtatta ccgcctttga gtgagctgat accgctcgcc gcagccgaac 3480
gaccgagcgc agcgagtcag tgagcgagga agcggaagag cgcccaatac gcaaaccgcc 3540

tctccccgcg cgttggccga ttcattaatg cagctggcac gacaggtttc ccgactggaa 3600
agcgggcagt gagcgcaacg caattaatgt gagttagctc actcattagg caccccaggc 3660
tttacacttt atgcttccgg ctcgtatgtt gtgtggaatt gtgagcggat aacaatttca 3720
cacaggaaac agctatgacc atgattacga attgcctgca ggcagctgcg cgctcgctcg 3780
ctcactgagg ccgcccgggc aaagcccggg cgtcgggcga cctttggtcg cccggcctca 3840
gtgagcgagc gagcgcgcag agagggagtg gccaactcca tcactagggg ttcctatcga 3900
tatcaagctt taatagtaat caattacggg gtcattagtt catagcccat atatggagtt 3960
ccgcgttaca taacttacgg taaatggccc gcctggctga ccgcccaacg acccccgccc 4020
attgacgtca ataatgacgt atgttcccat agtaacgcca atagggactt tccattgacg 4080
tcaatgggtg gagtatttac ggtaaactgc ccacttggca gtacatcaag tgtatcatat 4140
gccaagtacg ccccctattg acgtcaatga cggtaaatgg cccgcctggc attatgccca 4200
gtacatgacc ttatgggact ttcctacttg gcagtacatc tacgtattag tcatcgctat 4260
taccatggtg atgcggtttt ggcagtacat caatgggcgt ggatagcggt ttgactcacg 4320
gggatttcca agtctccacc ccattgacgt caatgggagt ttgttttggc accaaaatca 4380
acgggacttt ccaaaatgtc gtaacaactc cgccccattg acgcaaatgg gcggtaggcg 4440
tgtacggtgg gaggtctata taagcagagc tggtttagtg gatatcctta agggcccagc 4500
cggcctcgcg agaattctct agcaagctgt gaccggcgcc tacgctagac gccaccatgg 4560
agagcgacga gagcggcctg cccgccatgg agatcgagtg ccgcatcacc ggcaccctga 4620
acggcgtgga gttcgagctg gtgggcggcg gagagggcac ccccaagcag ggccgcatga 4680
ccaacaagat gaagagcacc aaaggcgccc tgaccttcag cccctacctg ctgagccacg 4740
tgatgggcta cggcttctac cacttcggca cctaccccag cggctacgag aaccccttcc 4800
tgcacgccat caacaacggc ggctacacca acacccgcat cgagaagtac gaggacggcg 4860
gcgtgctgca cgtgagcttc agctaccgct acgaggccgg ccgcgtgatc ggcgacttca 4920
aggtggtggg caccggcttc cccgaggaca gcgtgatctt caccgacaag atcatccgca 4980
gcaacgccac cgtggagcac ctgcacccca tgggcgataa cgtgctggtg ggcagcttcg 5040
cccgcacctt cagcctgcgc gacggcggct actacagctt cgtggtggac agccacatgc 5100
acttcaagag cgccatccac cccagcatcc tgcagaacgg gggccccatg ttcgccttcc 5160
gccgcgtgga ggagctgcac agcaacaccg agctgggcat cgtggagtac cagcacgcct 5220
tcaagacccc catcgccttc gccagatccc gcgctcagtc gtccaattct gccgtggacg 5280
gcaccgccgg acccggctcc accggatctc gcgagggcag aggaagtctt ctaacatgcg 5340

gtgacgtgga ggagaatccc ggccctatga ccgagtacaa gcccacggtg cgcctcgcca 5400

cccgcgacga cgtccccagg gccgtacgca ccctcgccgc cgcgttcgcc gactaccccg 5460

ccacgcgcca caccgtcgat ccggaccgcc acatcgagcg ggtcaccgag ctgcaagaac 5520

tcttcctcac gcgcgtcggg ctcgacatcg gcaaggtgtg ggtcgcggac gacggcgccg 5580

cggtggcggt ctggaccacg ccggagagcg tcgaagcggg ggcggtgttc gccgagatcg 5640

gcccgcgcat ggccgagttg agcggttccc ggctggccgc gcagcaacag atggaaggcc 5700

tcctggcgcc gcaccggccc aaggagcccg cgtggttcct ggccaccgtc ggcgtctcgc 5760

ccgaccacca gggcaagggt ctgggcagcg ccgtcgtgct ccccggagtg gaggcggccg 5820

agcgcgccgg ggtgcccgcc ttcctggaga cctccgcgcc ccgcaacctc cccttctacg 5880

agcggctcgg cttcaccgtc accgccgacg tcgaggtgcc cgaaggaccg cgcacctggt 5940

gcatgacccg caagcccggt gcctgaaatc aacctctgga ttacaaaatt tgtgaaagat 6000

tgactggtat tcttaactat gttgctcctt ttacgctatg tggatacgct gctttaatgc 6060

ctttgtatca gtt 6073

(SEQ ID NO: 2)

atgactctct taaggtagcc aaatttccat aggctccgcc cccctgacga gcatcacaaa 60
aatcgacgct caagtcagag gtggcgaaac ccgacaggac tataaagata ccaggcgttt 120
ccccctggaa gctccctcgt gcgctctcct gttccgaccc tgccgcttac cggatacctg 180
tccgcctttc tcccttcggg aagcgtggcg ctttctcata gctcacgctg taggtatctc 240
agttcggtgt aggtcgttcg ctccaagctg ggctgtgtgc acgaaccccc cgttcagccc 300
gaccgctgcg ccttatccgg taactatcgt cttgagtcca acccggtaag acacgactta 360
tcgccactgg cagcagccac tggtaacagg attagcagag cgaggtatgt aggcggtgct 420
acagagttct tgaagtggtg gcctaactac ggctacacta gaaggacagt atttggtatc 480
tgcgctctgc tgaagccagt taccttcgga aaaagagttg gtagctcttg atccggcaaa 540
caaaccaccg ctggtagcgg tggttttttt gtttgcaagc agcagattac gcgcagaaaa 600
aaaggatctc aagaagatcc tttgatcttt tctacggggt ctgacgctca gtggaacgaa 660
aactcacgtt aagggatttt ggtcatgaga ttatcaaaaa ggatcttcac ctagatcctt 720
ttaaattaaa aatgaagttt taaatcaatc taaagtatat atgagtaaac ttggtctgac 780
agttaccaat gcttaatcag tgaggcacct atctcagcga tctgtctatt tcgttcatcc 840
atagttgcct gactccccgt cgtgtagata actacgatac gggagggctt accatctggc 900
cccagtgctg caatgatacc gcgagaccca cgctcaccgg ctccagattt atcagcaata 960
aaccagccag ccggaagggc cgagcgcaga agtggtcctg caactttatc cgcctccatc 1020
cagtctatta attgttgccg ggaagctaga gtaagtagtt cgccagttaa tagtttgcgc 1080
aacgttgttg ccattgctac aggcatcgtg gtgtcacgct cgtcgtttgg tatggcttca 1140
ttcagctccg gttcccaacg atcaaggcga gttacatgat cccccatgtt gtgcaaaaaa 1200
gcggttagct ccttcggtcc tccgatcgtt gtcagaagta agttggccgc agtgttatca 1260
ctcatggtta tggcagcact gcataattct cttactgtca tgccatccgt aagatgcttt 1320
tctgtgactg gtgagtactc aaccaagtca ttctgagaat agtgtatgcg gcgaccgagt 1380
tgctcttgcc cggcgtcaat acgggataat accgcgccac atagcagaac tttaaaagtg 1440
ctcatcattg gaaaacgttc ttcggggcga aaactctcaa ggatcttacc gctgttgaga 1500
tccagttcga tgtaacccac tcgtgcaccc aactgatctt cagcatcttt tactttcacc 1560
agcgtttctg ggtgagcaaa aacaggaagg caaaatgccg caaaaaaggg aataagggcg 1620
acacggaaat gttgaatact catactcttc ctttttcaat attattgaag catttatcag 1680
ggttattgtc tcatgagcgg atacatattt gaatgtattt agaaaaataa acaaataggg 1740

gttccgcgca catttccccg aaaagtgcca cctaaattgt aagcgttaat attttgttaa 1800

aattcgcgtt aaatttttgt taaatcagct cattttttaa ccaataggcc gaaatcggca 1860

aaatccctta taaatcaaaa gaatagaccg agatagggtt gagtgttgtt ccagtttgga 1920

acaagagtcc actattaaag aacgtggact ccaacgtcaa agggcgaaaa accgtctatc 1980

agggcgatgg cccactacgt gaaccatcac cctaatcaag ttttttgggg tcgaggtgcc 2040

gtaaagcact aaatcggaac cctaaaggga gcccccgatt tagagcttga cggggaaagc 2100

cggcgaacgt ggcgagaaag gaagggaaga aagcgaaagg agcgggcgct agggcgctgg 2160

caagtgtagc ggtcacgctg cgcgtaacca ccacacccgc cgcgcttaat gcgccgctac 2220

agggcgcgtc ccattcgcca ttcaggctgc gcaactgttg ggaagggcga tcggtgcggg 2280

cctcttcgct attacgccag ctggcgaaag ggggatgtgc tgcaaggcga ttaagttggg 2340

taacgccagg gttttcccag tcacgacgtt gttttatcgg tctgtatatc gaggtttatt 2400

tattaatttg aatagatatt aagttttatt atatttacac ttacatacta ataataaatt 2460

caacaaacaa tttatttatg tttatttatt tattaaaaaa aaacaaaaac tcaaaatttc 2520

ttctataaag taacaaaact tttatcgaat tcctgcagcc cgggggatcc actagttcta 2580

gagggacagc ccccccccaa agcccccagg gatgtaatta cgtccctccc ccgctagggg 2640

gcagcagcga gccgcccggg gctccgctcc ggtccggcgc tcccccgca tccccgagcc 2700

ggcagcgtgc ggggacagcc cgggcacggg gaaggtggca cgggatcgct ttcctctgaa 2760

cgcttctcgc tgctctttga gcctgcagac acctgggggg atacggggaa aaggcctcca 2820

aggccagctt cccacaataa gttgggtgaa ttttggctca ttcctccttt ctataggatt 2880

gaggtcagag ctttgtgatg ggaattctgt ggaatgtgtg tcagttaggg tgtggaaagt 2940

cccgacattg attattgact agttattaat agtaatcaat tacggggtca ttagttcata 3000

gcccatatat ggagttccgc gttacataac ttacggtaaa tggcccgcct ggctgaccgc 3060

ccaacgaccc ccgcccattg acgtcaataa tgacgtatgt tcccatagta acgccaatag 3120

ggactttcca ttgacgtcaa tgggtggagt atttacggta aactgcccac ttggcagtac 3180

atcaagtgta tcatatgcca agtacgcccc ctattgacgt caatgacggt aaatggcccg 3240

cctggcatta tgcccagtac atgaccttat gggactttcc tacttggcag tacatctacg 3300

tattagtcat cgctattacc atggtgatgc ggttttggca gtacatcaat gggcgtggat 3360

agcggtttga ctcacgggga tttccaagtc tccaccccat tgacgtcaat gggagtttgt 3420

tttggcacca aaatcaacgg gactttccaa aatgtcgtaa caactccgcc ccattgacgc 3480

aaatgggcgg taggcgtgta cggtgggagg tctatataag cagagctggt ttagtgaacc 3540

gtcagatcag atctttgtcg atcctaccat ccactcgaca cacccgccag cggccgcagt 3600

tgcgcagcca tcgacgtcag acgcggaagc ttcgatcaac tacgcagaca ggtaccaaaa 3660

caaatgttct cgtcacgtgg gcatgaatct gatgctgttt ccctgcagac aatgcgagag 3720

aatgaatcag aattcaaata tctgcttcac tcacggacag aaagactgtt tagagtgctt 3780

tcccgtgtca gaatctcaac ccgtttctgt cgtcaaaaag gcgtatcaga aactgtgcta 3840

cattcatcat atcatgggaa aggtgccaga cgcttgcact gcctgcgatc tggtcaatgt 3900

ggatttggat gactgcatct ttgaacaata aatgatttaa atcaggtatg tcttttgttg 3960

atcaccctcc agattggttg gaagaagttg gtgaaggtct tcgcgagttt ttgggccttg 4020

aagcgggccc accgaaacca aaacccaatc agcagcatca agatcaagcc cgtggtcttg 4080

tgctgcctgg ttataactat ctcggacccg gaaacggtct cgatcgagga gagcctgtca 4140

acagggcaga cgaggtcgcg cgagagcacg acatctcgta caacgagcag cttgaggcgg 4200

gagacaaccc ctacctcaag tacaaccacg cggacgccga gtttcaggag aagctcgccg 4260

acgacacatc cttcggggga aacctcggaa aggcagtctt tcaggccaag aaaagggttc 4320

tcgaaccttt tggcctggtt gaagagggtg ctaagacggc ccctaccgga aagcggatag 4380

acgaccactt tccaaaaaga aagaaggctc ggaccgaaga ggactccaag ccttccacct 4440

cgtcagacgc cgaagctgga cccagcggat cccagcagct gcaaatccca gcccaaccag 4500

cctcaagttt gggagctgat acaatgtctg cgggaggtgg cggcccattg ggcgacaata 4560

accaaggtgc cgatggagtg ggcaatgcct cgggagattg gcattgcgat tccacgtgga 4620

tgggggacag agtcgtcacc aagtccaccc gaacctgggt gctgcccagc tacaacaacc 4680

accagtaccg agagatcaaa agcggctccg tcgacggaag caacgccaac gcctactttg 4740

gatacagcac cccctggggg tactttgact ttaaccgctt ccacagccac tggagccccc 4800

gagactggca aagactcatc aacaactact ggggcttcag accccggtcc ctcagagtca 4860

aaatcttcaa cattcaagtc aaagaggtca cggtgcagga ctccaccacc accatcgcca 4920

acaacctcac ctccaccgtc caagtgttta cggacgacga ctaccagctg ccctacgtcg 4980

tcggcaacgg gaccgaggga tgcctgccgg ccttccctcc gcaggtcttt acgctgccgc 5040

agtacggtta cgcgacgctg aaccgcgaca acacagaaaa tcccaccgag aggagcagct 5100

tcttctgcct agagtacttt cccagcaaga tgctgagaac gggcaacaac tttgagttta 5160

cctacaactt tgaggaggtg cccttccact ccagcttcgc tcccagtcag aacctgttca 5220

agctggccaa cccgctggtg gaccagtact tgtaccgctt cgtgagcaca aataacactg 5280

gcggagtcca gttcaacaag aacctggccg ggagatacgc caacacctac aaaaactggt 5340

tcccggggcc catgggccga acccagggct ggaacctggg ctccggggtc aaccgcgcca 5400
gtgtcagcgc cttcgccacg accaatagga tggagctcga gggcgcgagt taccaggtgc 5460
ccccgcagcc gaacggcatg accaacaacc tccagggcag caacacctat gccctggaga 5520
acactatgat cttcaacagc cagccggcga acccgggcac caccgccacg tacctcgagg 5580
gcaacatgct catcaccagc gagagcgaga cgcagccggt gaaccgcgtg gcgtacaacg 5640
tcggcgggca gatggccacc aacaaccaga gctccaccac tgcccccgcg accggcacgt 5700
acaacctcca ggaaatcgtg cccggcagcg tgtggatgga gagggacgtg tacctccaag 5760
gacccatctg ggccaagatc ccagagacgg ggcgcactt tcacccctct ccggccatgg 5820
gcggattcgg actcaaacac ccaccgccca tgatgctcat caagaacacg cctgtgcccg 5880
gaaatatcac cagcttctcg gacgtgcccg tcagcagctt catcacccag tacagcaccg 5940
ggcaggtcac cgtggagatg gagtgggagc tcaagaagga aaactccaag aggtggaacc 6000
cagagatcca gtacacaaac aactacaacg acccccagtt tgtggacttt gccccggaca 6060
gcaccgggga atacagaacc accagaccta tcggaacccg ataccttacc cgacccettt 6120
aacccgggag ttctagggat ctggcggccg ctcgagtcta gagggcccgt ttaaacccgc 6180
tgatcagcct cgactgtgcc ttctagttgc cagccatctg ttgtttgccc ctcccccgtg 6240
ccttccttga ccctggaagg tgccactccc actgtccttt cctaataaaa tgaggaaatt 6300
gcatcgcatt gtctgagtag gtgtcattct attctggggg gtggggtggg gcaggacagc 6360
aagggggagg attgggaaga caatagcagg catgctgggg atgcggtggg ctctatgggc 6420
gatcgctagc gtttaaactt aagcttggta ccgagctcgg atccactagt ccagtgtggt 6480
ggaattcctg cttcgcgatg tacgggccag atatacgcgt tgtttactca tgtctggggg 6540
tgggcgaaga actccagcat gagatccccg cgctggagga tcatccagcc ggcgtcccgg 6600
aaaacgattc cgaagcccaa cctttgcctg caggtcgact ctagaggatc cgaaaaaacc 6660
tcccacacct ccccctgaac ctgaaacata aaatgaatgc aattgttgtt gttaacttgt 6720
ttattgcagc ttataatggt tacaaataaa gcaatagcat cacaaatttc acaaataaag 6780
catttttttc actgcattct agttgtggtt tgtccaaact catcaatgta tcttatcatg 6840
tctggatccc cgcggccgcg gtacccacgt gggcgcgcca tttaaatgat aactcagaga 6900
gagtgtcctc gagccaatct ggaagataac catcggcagc catacctgat ttaaatcatt 6960
tattgttcaa agatgcagtc atccaaatcc acattgacca gatcgcaggc agtgcaagcg 7020
tctggcacct ttcccatgat atgatgaatg tagcacagtt tctgatacgc ctttttgacg 7080
acagaaacgg gttgagattc tgacacggga aagcactcta aacagtcttt ctgtccgtga 7140

gtgaagcaga tatttgaatt ctgattcatt ctctcgcatt gtctgcaggg aaacagcatc 7200
agattcatgc ccacgtgacg agaacatttg ttttggtacc tgtctgcgta gttgatcgaa 7260
gcttccgcgt ctgacgtcga tggctgcgca actgactcgc gcacccgttt gggctcactt 7320
atatctgcgt cactgggggc gggtcttttc ttggctccac ccttttttgac gtagaattca 7380
tgctccacct caaccacgtg atcctttgcc caccggaaaa agtctttgac ttcctgcttg 7440
gtgaccttcc caaagtcatg atccagacgg cgggtgagtt caaatttgaa catccggtct 7500
tgcaacggct gctggtgttc gaaggtcgtt gagttcccgt caatcacggc gcacatgttg 7560
gtgttggagg tgacgatcac gggagtcggg tctatctggg ccgaggactt gcatttctgg 7620
tccacgcgca ccttgcttcc tccgagaatg gctttggccg actccacgac cttggcggtc 7680
atcttcccct cctcccacca gatcaccatc ttgtcgacac agtcgttgaa gggaaagttc 7740
tcattggtcc agtttacgca cccgtagaag ggcacagtgt gggctatggc ctccgcgatg 7800
ttggtcttcc cggtagttgc aggcccaaac agccagatgg tgttcctctt gccgaacttt 7860
ttcgtggccc atcccagaaa gacggaagcc gcatattggg gatcgtaccc gtttagttcc 7920
aaaattttat aaatccgatt gctggaaatg tcctccacgg gctgctggcc caccaggtag 7980
tcgggggcgg ttttagtcag gctcataatc tttcccgcat tgtccaaggc agccttgatt 8040
tgggaccgcg agttggaggc cgcattgaag gagatgtatg aggcctggtc ctcctggatc 8100
cactgcttct ccgaggtaat ccccttgtcc acgagccacc cgaccagctc catgtacctg 8160
gctgaagttt ttgatctgat caccggcgca tcagaattgg gattctgatt ctctttgttc 8220
tgctcctgcg tctgcgacac gtgcgtcaga tgctgcgcca ccaaccgttt acgctccgtg 8280
agattcaaac aggcgcttaa atactgttcc atattagtcc acgcccactg gagctcaggc 8340
tgggttttgg ggagcaagta attggggatg tagcactcat ccaccacctt gttcccgcct 8400
ccggcgccat ttctggtctt tgtgaccgcg aaccagtttg gcaaagtcgg ctcgatcccg 8460
cggtaaattc tctgaatcag tttttcgcga atctgactca ggaaacgtcc caaaaccatg 8520
gatttcaccc cggtggtttc cacgagcacg tgcatgtgga agtagctctc tcccttctca 8580
aattgcacaa agaaaagggc ctccggggcc ttactcacac ggcgccattc cgtcagaaag 8640
tcgcgctgca gcttctcggc cacggtcagg ggtgcctgct caatcagatt cagatccatg 8700
tcagaatctg gcggcaactc ccattccttc tcggccaccc agttcacaaa gctgtcagaa 8760
atgccgggca gatgctcgtc aaggtcgctg gggaccttaa tcacaatctc gtaaaacccc 8820
ggcatggcgg cgataactat tatcatcgtg tttttcaaag gaaaaccacg tccccgtggt 8880
tcgggggggcc tagacgtttt tttaacctcg actaaacaca tgtaaagcat gtgcaccgag 8940

gccccagatc agatcccata caatggggta ccttctgggc atccttcagc cccttgttga 9000
atacgcttga ggagagccat ttgactcttt ccacaactat ccaactcaca acgtggcact 9060
ggggttgtgc cgcctttgca ggtgtatctt atacacgtgg cttttggccg cagaggcacc 9120
tgtcgccagg tggggggttc cgctgcctgc aaagggtcgc tacagacgtt gtttgtcttc 9180
aagaagcttc cagaggaact gcttccttca cgacattcaa cagaccttgc attcctttgg 9240
cgagagggga aagaccccta ggaatgctcg tcaagaagac agggccaggt ttccgggccc 9300
tcacattgcc aaaagacggc aatatggtgg aaaataacat atagacaaac gcacaccggc 9360
cttattccaa gcggcttcgg ccagtaacgt taggggggggg gggcggaatt ctatccctca 9420
gttatctcag agagagtgtc ctcgagccaa tctggaagat aaccatcggc agccatacct 9480
gatttaaatc atttattgtt caaagatgca gtcatccaaa tccacattga ccagatcgca 9540
ggcagtgcaa gcgtctggca cctttcccat gatatgatga atgtagcaca gtttctgata 9600
cgcctttttg acgacagaaa cgggttgaga ttctgacacg ggaaagcact ctaaacagtc 9660
tttctgtccg tgagtgaagc agatatttga attctgattc attctctcgc attgtctgca 9720
gggaaacagc atcagattca tgcccacgtg acgagaacat ttgttttggt acctgtctgc 9780
gtagttgatc gaagcttccg cgtctgacgt cgatggctgc gcaactgact cgcgcacccg 9840
tttgggctca cttatatctg cgtcactggg ggcgggtctt ttcttggctc cacccttttt 9900
gacgtagaat tcatgctcca cctcaaccac gtgatccttt gcccaccgga aaaagtcttt 9960
gacttcctgc ttggtgacct tcccaaagtc atgatccaga cggcgggtga gttcaaattt 10020
gaacatccgg tcttgcaacg gctgctggtg ttcgaaggtc gttgagttcc cgtcaatcac 10080
ggcgcacatg ttggtgttgg aggtgacgat cacgggagtc gggtctatct gggccgagga 10140
cttgcatttc tggtccacgc gcaccttgct tcctccgaga atggctttgg ccgactccac 10200
gaccttggcg gtcatcttcc cctcctccca ccagatcacc atcttgtcga cacagtcgtt 10260
gaagggaaag ttctcattgg tccagtttac gcacccgtag aagggcacag tgtgggctat 10320
ggcctccgcg atgttggtct tcccggtagt tgcaggccca aacagccaga tggtgttcct 10380
cttgccgaac tttttcgtgg cccatcccag aaagacggaa gccgcatatt ggggatcgta 10440
cccgtttagt tccaaaattt tataaatccg attgctggaa atgtcctcca cgggctgctg 10500
gcccaccagg tagtcggggg cggttttagt caggctcata atctttcccg cattgtccaa 10560
ggcagccttg atttgggacc gcgagttgga ggccgcattg aaggagatgt atgaggcctg 10620
gtcctcctgg atccactgct tctccgaggt aatccccttg tccacgagcc acccgaccag 10680
ctccatggtg gcttcggggc cgcggaggct ggatcggtcc cggtgtcttc tatggaggtc 10740

aaaacagcgt ggatggcgtc tccaggcgat ctgacggttc actaaacgag ctctgcttat 10800

atagacctcc caccgtacac gcctaccgcc catttgcgtc aatggggcgg agttgttacg 10860

acattttgga aagtcccgtt gattttggtg ccaaaacaaa ctcccattga cgtcaatggg 10920

gtggagactt ggaaatcccc gtgagtcaaa ccgctatcca cgcccattga tgtactgcca 10980

aaaccgcatc accatggtaa tagcgatgac taatacgtag atgtactgcc aagtaggaaa 11040

gtcccataag gtcatgtact gggcataatg ccaggcgggc catttaccgt cattgacgtc 11100

aatagggggc gtacttggca tatgatacac ttgatgtact gccaagtggg cagtttaccg 11160

taaatactcc acccattgac gtcaatggaa agtccctatt ggcgttacta tgggaacata 11220

cgtcattatt gacgtcaatg ggcgggggtc gttgggcggt cagccaggcg ggccatttac 11280

cgtaagttat gtaacgcgga actccatata tgggctatga actaatgacc ccgtaattga 11340

ttactattaa taactagtca ataatcaatg tcttacatcc ctgggggctt tggggggggg 11400

ctgtccctct agagcggccg ccaccgcggt ggagctccag cttttgttcc ctttagtgag 11460

ggttaattag atcttaatac gactcactat agggcgaatt gggtaccggg ccccccctcg 11520

aggtcgacgg tatcgtgtga aattgttatc cgctcacaat tccacacaac atacgagccg 11580

gaagcataaa gtgtaaagcc tggggtgcct aatgagtgag ctaactcaca ttaattgcgt 11640

tgcgctcact gcccgctttc cagtcgggaa acctgtcgtg ccagctgcat taatgaatcg 11700

gccaacgcgc ggggagaggc ggtttgcgta ttgggcgctc ttccgcttcc tcgctcactg 11760

actcgctgcg ctcggtcgtt cggctgcggc gagcggtatc agctcactca aaggcggtaa 11820

tacggttatc cacagaatca ggggataacg caggaaagaa catgtgagca aaaggccagc 11880

aaaaggccag gaaccgtaaa aaggccgcgt tgctggcgtt 11920

(SEQ ID NO: 3)

ggtacccaac tccatgctta acagtcccca ggtacagccc accctgcgtc gcaaccagga 60
acagctctac agcttcctgg agcgccactc gccctacttc cgcagccaca gtgcgcagat 120
taggagcgcc acttcttttt gtcacttgaa aaacatgtaa aaataatgta ctaggagaca 180
ctttcaataa aggcaaatgt ttttatttgt acactctcgg gtgattattt accccccacc 240
cttgccgtct gcgccgttta aaaatcaaag gggttctgcc gcgcatcgct atgcgccact 300
ggcagggaca cgttgcgata ctggtgttta gtgctccact taaactcagg cacaaccatc 360
cgcggcagct cggtgaagtt ttcactccac aggctgcgca ccatcaccaa cgcgtttagc 420
aggtcgggcg ccgatatctt gaagtcgcag ttggggcctc cgccctgcgc gcgcgagttg 480
cgatacacag ggttgcagca ctggaacact atcagcgccg ggtggtgcac gctggccagc 540
acgctcttgt cggagatcag atccgcgtcc aggtcctccg cgttgctcag ggcgaacgga 600
gtcaactttg gtagctgcct tcccaaaaag ggtgcatgcc caggctttga gttgcactcg 660
caccgtagtg gcatcagaag gtgaccgtgc ccggtctggg cgttaggata cagcgcctgc 720
atgaaagcct tgatctgctt aaaagccacc tgagcctttg cgccttcaga gaagaacatg 780
ccgcaagact tgccggaaaa ctgattggcc ggacaggccg cgtcatgcac gcagcacctt 840
gcgtcggtgt tggagatctg caccacattt cggccccacc ggttcttcac gatcttggcc 900
ttgctagact gctccttcag cgcgcgctgc ccgttttcgc tcgtcacatc catttcaatc 960
acgtgctcct tatttatcat aatgctcccg tgtagacact taagctcgcc ttcgatctca 1020
gcgcagcggt gcagccacaa cgcgcagccc gtgggctcgt ggtgcttgta ggttacctct 1080
gcaaacgact gcaggtacgc ctgcaggaat cgccccatca tcgtcacaaa ggtcttgttg 1140
ctggtgaagg tcagctgcaa cccgcggtgc tcctcgttta gccaggtctt gcatacggcc 1200
gccagagctt ccacttggtc aggcagtagc ttgaagtttg cctttagatc gttatccacg 1260
tggtacttgt ccatcaacgc gcgcgcagcc tccatgccct tctcccacgc agacacgatc 1320
ggcaggctca gcgggtttat caccgtgctt tcactttccg cttcactgga ctcttccttt 1380
tcctcttgcg tccgcatacc ccgcgccact gggtcgtctt cattcagccg ccgcaccgtg 1440
cgcttacctc ccttgccgtg cttgattagc accggtgggt tgctgaaacc caccatttgt 1500
agcgccacat cttctctttc ttcctcgctg tccacgatca cctctgggga tggcgggcgc 1560
tcgggcttgg gagaggggcg cttcttttc tttttggacg caatggccaa atccgccgtc 1620
gaggtcgatg gccgcgggct gggtgtgcgc ggcaccagcg catcttgtga cgagtcttct 1680
tcgtcctcgg actcgagacg ccgcctcagc cgcttttttg ggggcgcgcg ggaggcggc 1740

ggcgacggcg acggggacga cacgtcctcc atggttggtg gacgtcgcgc cgcaccgcgt 1800

ccgcgctcgg gggtggtttc gcgctgctcc tcttcccgac tggccatttc cttctcctat 1860

aggcagaaaa agatcatgga gtcagtcgag aaggaggaca gcctaaccgc cccctttgag 1920

ttcgccacca ccgcctccac cgatgccgcc aacgcgccta ccaccttccc cgtcgaggca 1980

cccccgcttg aggaggagga agtgattatc gagcaggacc caggttttgt aagcgaagac 2040

gacgaggatc gctcagtacc aacagaggat aaaaagcaag accaggacga cgcagaggca 2100

aacgaggaac aagtcgggcg gggggaccaa aggcatggcg actacctaga tgtgggagac 2160

gacgtgctgt tgaagcatct gcagcgccag tgcgccatta tctgcgacgc gttgcaagag 2220

cgcagcgatg tgcccctcgc catagcggat gtcagccttg cctacgaacg ccacctgttc 2280

tcaccgcgcg taccccccaa acgccaagaa aacggcacat gcgagcccaa cccgcgcctc 2340

aacttctacc ccgtatttgc cgtgccagag gtgcttgcca cctatcacat ctttttccaa 2400

aactgcaaga taccccctatc ctgccgtgcc aaccgcagcc gagcggacaa gcagctggcc 2460

ttgcggcagg gcgctgtcat acctgatatc gcctcgctcg acgaagtgcc aaaaatcttt 2520

gagggtcttg gacgcgacga gaaacgcgcg gcaaacgctc tgcaacaaga aaacagcgaa 2580

aatgaaagtc actgtggagt gctggtggaa cttgagggtg acaacgcgcg cctagccgtg 2640

ctgaaacgca gcatcgaggt cacccacttt gcctacccgg cacttaacct accccccaag 2700

gttatgagca cagtcatgag cgagctgatc gtgcgccgtg cacgaccccct ggagagggat 2760

gcaaacttgc aagaacaaac cgaggagggc ctacccgcag ttggcgatga gcagctggcg 2820

cgctggcttg agacgcgcga gcctgccgac ttggaggagc gacgcaagct aatgatggcc 2880

gcagtgcttg ttaccgtgga gcttgagtgc atgcagcggt tctttgctga cccggagatg 2940

cagcgcaagc tagaggaaac gttgcactac acctttcgcc agggctacgt gcgccaggcc 3000

tgcaaaattt ccaacgtgga gctctgcaac ctggtctcct accttggaat tttgcacgaa 3060

aaccgcctcg ggcaaaacgt gcttcattcc acgctcaagg gcgaggcgcg ccgcgactac 3120

gtccgcgact gcgtttactt atttctgtgc tacacctggc aaacggccat gggcgtgtgg 3180

cagcaatgcc tggaggagcg caacctaaag gagctgcaga agctgctaaa gcaaaacttg 3240

aaggacctat ggacggcctt caacgagcgc tccgtggccg cgcacctggc ggacattatc 3300

ttccccgaac gcctgcttaa aaccctgcaa cagggtctgc cagacttcac cagtcaaagc 3360

atgttgcaaa actttaggaa ctttatccta gagcgttcag gaattctgcc cgccacctgc 3420

tgtgcgcttc ctagcgactt tgtgcccatt aagtaccgtg aatgccctcc gccgctttgg 3480

ggtcactgct accttctgca gctagccaac taccttgcct accactccga catcatggaa 3540

gacgtgagcg gtgacggcct actggagtgt cactgtcgct gcaacctatg caccccgcac 3600
cgctccctgg tctgcaattc gcaactgctt agcgaaagtc aaattatcgg tacctttgag 3660
ctgcagggtc cctcgcctga cgaaaagtcc gcggctccgg ggttgaaact cactccgggg 3720
ctgtggacgt cggcttacct tcgcaaattt gtacctgagg actaccacgc ccacgagatt 3780
aggttctacg aagaccaatc ccgcccgcca aatgcggagc ttaccgcctg cgtcattacc 3840
cagggccaca tccttggcca attgcaagcc atcaacaaag cccgccaaga gtttctgcta 3900
cgaaagggac ggggggttta cctggacccc cagtccggcg aggagctcaa cccaatcccc 3960
ccgccgccgc agccctatca gcagccgcgg gcccttgctt cccaggatgg cacccaaaaa 4020
gaagctgcag ctgccgccgc cgccacccac ggacgaggag gaatactggg acagtcaggc 4080
agaggaggtt ttggacgagg aggaggagat gatggaagac tgggacagcc tagacgaagc 4140
ttccgaggcc gaagaggtgt cagacgaaac accgtcaccc tcggtcgcat tcccctcgcc 4200
ggcgccccag aaattggcaa ccgttcccag catcgctaca acctccgctc ctcaggcgcc 4260
gccggcactg cctgttcgcc gacccaaccg tagatgggac accactggaa ccagggccgg 4320
taagtctaag cagccgccgc cgttagccca agagcaacaa cagcgccaag gctaccgctc 4380
gtggcgcggg cacaagaacg ccatagttgc ttgcttgcaa gactgtgggg gcaacatctc 4440
cttcgcccgc cgctttcttc tctaccatca cggcgtggcc ttcccccgta acatcctgca 4500
ttactaccgt catctctaca gcccctactg caccggcggc agcggcagcg gcagcaacag 4560
cagcggtcac acagaagcaa aggcgaccgg atagcaagac tctgacaaag cccaagaaat 4620
ccacagcggc ggcagcagca ggaggaggag cgctgcgtct ggcgcccaac gaacccgtat 4680
cgacccgcga gcttagaaat aggatttttc ccactctgta tgctatattt caacaaagca 4740
ggggccaaga acaagagctg aaaataaaaa acaggtctct gcgctccctc acccgcagct 4800
gcctgtatca caaaagcgaa gatcagcttc ggcgcacgct ggaagacgcg gaggctctct 4860
tcagcaaata ctgcgcgctg actcttaagg actagtttcg cgccctttct caaatttaag 4920
cgcgaaaact acgtcatctc cagcggccac acccggcgcc agcacctgtc gtcagcgcca 4980
ttatgagcaa ggaaattccc acgccctaca tgtggagtta ccagccacaa atgggacttg 5040
cggctggagc tgcccaagac tactcaaccc gaataaacta catgagcgcg ggaccccaca 5100
tgatatcccg ggtcaacgga atccgcgccc accgaaaccg aattctcctc gaacaggcgg 5160
ctattaccac cacacctcgt aataacctta atccccgtag ttggcccgct gccctggtgt 5220
accaggaaag tcccgctccc accactgtgg tacttcccag agacgcccag gccgaagttc 5280
agatgactaa ctcaggggcg cagcttgcgg gcggctttcg tcacagggtg cggtcgcccg 5340

ggcgttttag ggcggagtaa cttgcatgta ttgggaattg tagtttttttt aaaatgggaa 5400
gtgacgtatc gtgggaaaac ggaagtgaag atttgaggaa gttgtgggtt ttttggcttt 5460
cgtttctggg cgtaggttcg cgtgcggttt tctgggtgtt ttttgtggac tttaaccgtt 5520
acgtcatttt ttagtcctat atatactcgc tctgtacttg gcccttttta cactgtgact 5580
gattgagctg gtgccgtgtc gagtggtgtt ttttaatagg tttttttact ggtaaggctg 5640
actgttatgg ctgccgctgt ggaagcgctg tatgttgttc tggagcggga gggtgctatt 5700
ttgcctaggc aggagggttt ttcaggtgtt tatgtgtttt tctctcctat taattttgtt 5760
atacctccta tgggggctgt aatgttgtct ctacgcctgc gggtatgtat tcccccgggc 5820
tatttcggtc gctttttagc actgaccgat gttaaccaac ctgatgtgtt taccgagtct 5880
tacattatga ctccggacat gaccgaggaa ctgtcggtgg tgctttttaa tcacggtgac 5940
cagttttttt acggtcacgc cggcatggcc gtagtccgtc ttatgcttat aagggttgtt 6000
tttcctgttg taagacaggc ttctaatgtt taaatgtttt tttttttgtt attttatttt 6060
gtgtttaatg caggaacccg cagacatgtt tgagagaaaa atggtgtctt tttctgtggt 6120
ggttccggaa cttacctgcc tttatctgca tgagcatgac tacgatgtgc ttgctttttt 6180
gcgcgaggct ttgcctgatt ttttgagcag caccttgcat tttatatcgc cgcccatgca 6240
acaagcttac ataggggcta cgctggttag catagctccg agtatgcgtg tcataatcag 6300
tgtgggttct tttgtcatgg ttcctggcgg ggaagtggcc gcgctggtcc gtgcagacct 6360
gcacgattat gttcagctgg ccctgcgaag ggacctacgg gatcgcggta tttttgttaa 6420
tgttccgctt ttgaatctta tacaggtctg tgaggaacct gaatttttgc aatcatgatt 6480
cgctgcttga ggctgaaggt ggagggcgct ctggagcaga tttttacaat ggccggactt 6540
aatattcggg atttgcttag agacatattg ataaggtggc gagatgaaaa ttatttgggc 6600
atggttgaag gtgctggaat gtttatagag gagattcacc ctgaagggtt tagcctttac 6660
gtccacttgg acgtgagggc agtttgcctt ttggaagcca ttgtgcaaca tcttacaaat 6720
gccattatct gttctttggc tgtagagttt gaccacgcca ccggagggga gcgcgttcac 6780
ttaatagatc ttcattttga ggttttggat aatcttttgg aataaaaaaa aaaaaacatg 6840
gttcttccag ctcttcccgc tcctcccgtg tgtgactcgc agaacgaatg tgtaggttgg 6900
ctgggtgtgg cttattctgc ggtggtggat gttatcaggg cagcggcgca tgaaggagtt 6960
tacatagaac ccgaagccag gggcgcctg gatgctttga gagagtggat atactacaac 7020
tactacacag agcgagctaa gcgacgagac cggagacgca gatctgtttg tcacgcccgc 7080
acctggtttt gcttcaggaa atatgactac gtccggcgtt ccatttggca tgacactacg 7140

accaacacga tctcggttgt ctcggcgcac tccgtacagt agggatcgcc tacctccttt 7200
tgagacagag acccgcgcta ccatactgga ggatcatccg ctgctgcccg aatgtaacac 7260
tttgacaatg cacaacgtga gttacgtgcg aggtcttccc tgcagtgtgg gatttacgct 7320
gattcaggaa tgggttgttc cctgggatat ggttctgacg cgggaggagc ttgtaatcct 7380
gaggaagtgt atgcacgtgt gcctgtgttg tgccaacatt gatatcatga cgagcatgat 7440
gatccatggt tacgagtcct gggctctcca ctgtcattgt tccagtcccg gttccctgca 7500
gtgcatagcc ggcgggcagg ttttggccag ctggtttagg atggtggtgg atggcgccat 7560
gtttaatcag aggtttatat ggtaccggga ggtggtgaat tacaacatgc caaaagaggt 7620
aatgtttatg tccagcgtgt ttatgagggg tcgccactta atctacctgc gcttgtggta 7680
tgatggccac gtgggttctg tggtccccgc catgagcttt ggatacagcg ccttgcactg 7740
tgggattttg aacaatattg tggtgctgtg ctgcagttac tgtgctgatt taagtgagat 7800
cagggtgcgc tgctgtgccc ggaggacaag gcgtctcatg ctgcgggcgg tgcgaatcat 7860
cgctgaggag accactgcca tgttgtattc ctgcaggacg gagcggcggc ggcagcagtt 7920
tattcgcgcg ctgctgcagc accaccgccc tatcctgatg cacgattatg actctacccc 7980
catgtaggcg tggacttccc cttcgccgcc cgttgagcaa ccgcaagttg gacagcagcc 8040
tgtggctcag cagctggaca gcgacatgaa cttaagcgag ctgcccgggg agtttattaa 8100
tatcactgat gagcgtttgg ctcgacagga aaccgtgtgg aatataacac ctaagaatat 8160
gtctgttacc catgatatga tgctttttaa ggccagccgg ggagaaagga ctgtgtactc 8220
tgtgtgttgg gagggaggtg gcaggttgaa tactagggtt ctgtgagttt gattaaggta 8280
cggtgatcaa tataagctat gtggtggtgg ggctatacta ctgaatgaaa aatgacttga 8340
aattttctgc aattgaaaaa taaacacgtt gaaacataac atgcaacagg ttcacgattc 8400
tttattcctg ggcaatgtag gagaaggtgt aagagttggt agcaaaagtt tcagtggtgt 8460
attttccact ttcccaggac catgtaaaag acatagagta agtgcttacc tcgctagttt 8520
ctgtggattc actagaatcg atgtaggatg ttgcccctcc tgacgcggta ggagaagggg 8580
agggtgccct gcatgtctgc cgctgctctt gctcttgccg ctgctgagga gggggcgca 8640
tctgccgcag caccggatgc atctgggaaa agcaaaaaag gggctcgtcc ctgtttccgg 8700
aggaatttgc aagcggggtc ttgcatgacg gggaggcaaa cccccgttcg ccgcagtccg 8760
gccggcccga gactcgaacc gggggtcctg cgactcaacc cttggaaaat aaccctccgg 8820
ctacagggag cgagccactt aatgctttcg ctttccagcc taaccgctta cgccgcgcgc 8880
ggccagtggc caaaaaagct agcgcagcag ccgccgcgcc tggaaggaag ccaaaaggag 8940

cgctcccccg ttgtctgacg tcgcacacct gggttcgaca cgcgggcggt aaccgcatgg 9000
atcacggcgg acggccggat ccggggttcg aaccccggtc gtccgccatg ataccccttgc 9060
gaatttatcc accagaccac ggaagagtgc ccgcttacag gctctccttt tgcacggtct 9120
agagcgtcaa cgactgcgca cgcctcaccg gccagagcgt cccgaccatg gagcactttt 9180
tgccgctgcg caacatctgg aaccgcgtcc gcgactttcc gcgcgcctcc accaccgccg 9240
ccggcatcac ctggatgtcc aggtacatct acggattacg tcgacgttta aaccatatga 9300
tcagctcact caaaggcggt aatacggtta tccacagaat caggggataa cgcaggaaag 9360
aacatgtgag caaaaggcca gcaaaaggcc aggaaccgta aaaaggccgc gttgctggcg 9420
tttttccata ggctccgccc ccctgacgag catcacaaaa atcgacgctc aagtcagagg 9480
tggcgaaacc cgacaggact ataaagatac caggcgtttc cccctggaag ctccctcgtg 9540
cgctctcctg ttccgaccct gccgcttacc ggatacctgt ccgcctttct cccttcggga 9600
agcgtggcgc tttctcatag ctcacgctgt aggtatctca gttcggtgta ggtcgttcgc 9660
tccaagctgg gctgtgtgca cgaacccccc gttcagcccg accgctgcgc cttatccggt 9720
aactatcgtc ttgagtccaa cccggtaaga cacgacttat cgccactggc agcagccact 9780
ggtaacagga ttagcagagc gaggtatgta ggcggtgcta cagagttctt gaagtggtgg 9840
cctaactacg gctacactag aagaacagta tttggtatct gcgctctgct gaagccagtt 9900
accttcggaa aaagagttgg tagctcttga tccggcaaac aaaccaccgc tggtagcggt 9960
ggtttttttg tttgcaagca gcagattacg cgcagaaaaa aaggatctca agaagatcct 10020
ttgatctttt ctacggggtc tgacgctcag tggaacgaaa actcacgtta agggattttg 10080
gtcatgagat tatcaaaaag gatcttcacc tagatccttt taaattaaaa atgaagtttt 10140
aaatcaatct aaagtatata tgagtaaact tggtctgaca gttaccaatg cttaatcagt 10200
gaggcaccta tctcagcgat ctgtctattt cgttcatcca tagttgcctg actccccgtc 10260
gtgtagataa ctacgatacg ggagggctta ccatctggcc ccagtgctgc aatgataccg 10320
cgagacccac gctcaccggc tccagattta tcagcaataa accagccagc cggaagggcc 10380
gagcgcagaa gtggtcctgc aactttatcc gcctccatcc agtctattaa ttgttgccgg 10440
gaagctagag taagtagttc gccagttaat agtttgcgca acgttgttgc cattgctaca 10500
ggcatcgtgg tgtcacgctc gtcgtttggt atggcttcat tcagctccgg ttcccaacga 10560
tcaaggcgag ttacatgatc ccccatgttg tgcaaaaaag cggttagctc cttcggtcct 10620
ccgatcgttg tcagaagtaa gttggccgca gtgttatcac tcatggttat ggcagcactg 10680
cataattctc ttactgtcat gccatccgta agatgctttt ctgtgactgg tgagtactca 10740

accaagtcat tctgagaata gtgtatgcgg cgaccgagtt gctcttgccc ggcgtcaata 10800

cgggataata ccgcgccaca tagcagaact ttaaaagtgc tcatcattgg aaaacgttct 10860

tcggggcgaa aactctcaag gatcttaccg ctgttgagat ccagttcgat gtaacccact 10920

cgtgcaccca actgatcttc agcatctttt actttcacca gcgtttctgg gtgagcaaaa 10980

acaggaaggc aaaatgccgc aaaaaaggga ataagggcga cacggaaatg ttgaatactc 11040

atactcttcc tttttcaata ttattgaagc atttatcagg gttattgtct catgagcgga 11100

tacatatttg aatgtattta gaaaaataaa caaatagggg ttccgcgcac atttccccga 11160

aaagtgccac ctaaattgta agcgttaata ttttgttaaa attcgcgtta aatttttgtt 11220

aaatcagctc attttttaac caataggccg aaatcggcaa aatcccttat aaatcaaaag 11280

aatagaccga gatagggttg agtgttgttc cagtttggaa caagagtcca ctattaaaga 11340

acgtggactc caacgtcaaa gggcgaaaaa ccgtctatca gggcgatggc ccactacgtg 11400

aaccatcacc ctaatcaagt tttttggggt cgaggtgccg taaagcacta aatcggaacc 11460

ctaaagggag cccccgattt agagcttgac ggggaaagcc ggcgaacgtg gcgagaaagg 11520

aagggaagaa agcgaaagga gcgggcgcta gggcgctggc aagtgtagcg gtcacgctgc 11580

gcgtaaccac cacacccgcc gcgcttaatg cgccgctaca gggcgcgatg gatcc 11635

Each of the above-described plasmid stock solutions (TE buffer) having a concentration of 2 mg/mL, which had been proliferated with Escherichia coli, were mixed at an equimolar ratio. These solutions were mixed such that the ratio of the mass (g) was the ratio of the plasmid size (base length: bp).

In the case of batch electroporation (BEP), the plasmid mixed solution was added to a cell suspension adjusted to $120 \times 10^6$ cells/mL to prepare cell-plasmid mixted solution having plasmid concentrations of 60, 150, and 375 $\mu$g/mL.

In the case of flow electroporation (FEP), as in the case of BEP or continuous mixing perfusion was performed. A cell suspension of $40 \times 10^6$ cells/mL withdrawn from the culture reactor was continuously mixed with the plasmid stock solution at a flow rate ratio of the cell suspension and the plasmid stock solution of 12.3:1, and a cell-plasmid mixed solution having a plasmid concentration of 150 $\mu$g/mL was continuously supplied (fed) to the downstream FEP unit.

(4) Electroporation and (5) main culture

<Case of batch electroporation>

Electroporation was performed under the conditions described in the following table.

Two sets of 2 mm gap aluminum electrode cuvettes filled with 0.2 mL of the cell-plasmid mixture were prepared.

A desired pulse was applied using an electroporator NEPA21 manufactured by NEPAGENE Co., Ltd. The mixed solution after EP was collected with a pipette and transferred to a 125 mL flask.

After allowing the cells to stand in an incubator at 8% $CO_2$ and 37°C for 30 minutes, 12 mL of Balan-CD-HEK293 medium was added thereto. The cells were returned to the incubator again and cultured at 8% $CO_2$, 37°C, and 150 rpm for 48 hours.

<Case of flow electroporation>

The cell plasmid mixed solution was filled in a 50 mL syringe.

20 mL of the mixed solution was fed at 86 mL/min to the FEP device shown in FIGS. 1 and 2, having an inter-electrode gap of 3 mm, a flow channel width of 20 mm, and an electrode length of 20 mm. In the case of the plasmid continuous mixing, the cell solution was fed at 79.55 mL/min, and the plasmid stock solution was fed at 6.45 mL/min.

A pulse voltage was applied to the FEP electrode, with a voltage amplitude of 412.5 V (electric field strength of 1375 V/cm), pulse width of 3.5 ms, and pulse period of 450 ms. Continuous EP was performed on cells flowing in the electrode pair such that, on average, one pulse was applied to each cell.

The liquid after EP was collected into a collection bottle. 1.5 mL of the collected EP liquid was transferred to a 125 mL flask. After allowing the cells to stand in an incubator at 8% $CO_2$ and 37°C for 30 minutes, 12 mL of Balan-CD-HEK293 medium was added thereto. The cells were returned to the incubator again and cultured at 8% $CO_2$, 37°C, and 150 rpm for 48 hours.

(6) AAV recovery and (7) analysis

<Measurement of titer>

2 mL of the cell suspension after culturing for 48 hours was collected in a 50 mL centrifuge tube. 20 μL of Triton X (final concentration of 0.1%) and $1.25 \times 10^{-5}$ U/cells of Benzonase were added to the above-described cell suspension, 20 μL of 200 mmol/L $MgCl_2$ (final concentration of 2 mmol/L) was added thereto, and the mixture was stirred at 37°C and 180 rpm to decompose the nucleic acid (cell-derived genome, plasmid) in the suspension. The mixture was centrifuged at 10,625 g for 10 minutes in a 1.5 mL tube, and the supernatant was recovered. The capsid of AAV was thermally decomposed by a heating treatment to extract the genome in AAV. The number of ITR sequences was measured by ddPCR, and the AAV titer (vg/mL) was calculated.

<Measurement of Full ratio>

AAV was extracted and purified using the AAVpro (registered trademark) Purification Kit (manufactured by Takara Bio Inc.). The adjusted liquid after purification was analyzed by using high performance liquid chromatography to measure the Full rate. An anion exchange column was used as a separation column, and a fluorescence detector (excitation: 280 nm, detection: 348 nm) was used for detection. The Full ratio was calculated from the area value of each of the Empty peak and the Full peak of the AAV.

The determination of the evaluation was performed according to the following criteria.

<Evaluation of CNET>

A: 2.0E+05 or more and 4.0E+05 or less
B: more than 4.0E+05 and 5.0E+05 or less
C: 1.0E+5 or more and less than 2.0E+05, or more than 5.0E+05 and less than 1.0E+6
D: 1.0E+4 or more and less than 1.0E+5, or 1.0E+6 or more and 1.0E+7 or less
E: less than 1.0E+4 or more than 1.0E+7

<Evaluation of titer>

A: 1.0E+11 vg/mL or more
B: 5.0E+10 vg/mL or more and less than 1.0E+11 vg/mL
C: 1.0E+10 vg/mL or more and less than 5.0E+10 vg/mL
D: 1.0E+9 vg/mL or more and less than 1.0E+10 vg/mL
E: less than 1.0E+9 vg/mL

<Evaluation of Full ratio>

A: 30% or more
B: 20% or more and less than 30%
C: 10% or more and less than 20%
D: 5% or more and less than 10%
E: less than 5%

<Overall evaluation>

A: a case where the evaluation of the titer is A to C and the evaluation of the Full ratio is A
B: a case where the evaluation of the titer is A to C and the evaluation of the Full ratio is B
C: a case where the evaluation of the titer is A to C and the evaluation of the Full ratio is C or D
D: a case where the evaluation of the titer is A to C and the evaluation of the Full rate is E
E: a case where the evaluation of the titer is D
F: a case where the evaluation of the titer is E

[Table 1]

| | Preculture (cell supply) | | | | Electroporation | | | | | | | | | | | | | | |
|---|---|---|---|---|---|---|---|---|---|---|---|---|---|---|---|---|---|---|---|
| | | | | | Cell | | | Plasmid | | | EP method | Perforation pulse | | | Electrophoresis pulse | | | CNET product | CNET evaluation |
| | Culture method | Cell | Cell concentration | Culture medium | Cell concentration | Buffer | Medium exchange | Concentration | Ratio | Nucleic acid/-cell | | Electric field | Pulse width | Number of pulses | Electric field | Pulse width | Number of pulses | | |
| | | | | | | | | C | | | | Eh | Th | Nh | El | Tl | Nl | | |
| | - | - | Mcells ml | - | Mcell ml | - | - | ug ml | - | pg/cell | - | V/cm | ms | - | V/cm | ms | - | *1 | - |
| Example 1 | Perfusion | HEK293 | 43 | BlanCD | 120 | BlanCD | Yes | 150 | 1:1:1 | 1.25 | BEP | 1375 | 0.1 | 1 | | | | 2.1E-04 | D |
| Example 2 | Perfusion | HEK293 | 43 | BlanCD | 120 | BlanCD | Yes | 150 | 1:1:1 | 1.25 | BEP | 1375 | 0.6 | 1 | | | | 1.2E-05 | C |
| Example 3 | Perfusion | HEK293 | 43 | BlanCD | 120 | BlanCD | Yes | 150 | 1:1:1 | 1.25 | BEP | 1375 | 1.2 | 1 | | | | 2.5E-05 | A |
| Example 4 | Perfusion | HEK293 | 43 | BlanCD | 120 | BlanCD | Yes | 150 | 1:1:1 | 1.25 | BEP | 1375 | 2.4 | 1 | | | | 5.0E-05 | B |
| Example 5 | Perfusion | HEK293 | 43 | BlanCD | 120 | BlanCD | Yes | 150 | 1:1:1 | 1.25 | BEP | 1375 | 3.6 | 1 | | | | 7.4E-05 | C |
| Example 6 | Perfusion | HEK293 | 43 | BlanCD | 120 | BlanCD | Yes | 150 | 1:1:1 | 1.25 | BEP | 1375 | 8 | 1 | | | | 1.7E-06 | D |
| Example 7 | Perfusion | HEK293 | 43 | BlanCD | 120 | BlanCD | Yes | 375 | 1:1:1 | 3.125 | BEP | 1375 | 0.25 | 1 | | | | 1.3E-05 | C |
| Example 8 | Perfusion | HEK293 | 43 | BlanCD | 120 | BlanCD | Yes | 375 | 1:1:1 | 3.125 | BEP | 1375 | 0.5 | 1 | | | | 2.6E-05 | A |
| Example 9 | Perfusion | HEK293 | 43 | BlanCD | 120 | BlanCD | Yes | 375 | 1:1:1 | 3.125 | BEP | 1375 | 1 | 1 | | | | 5.2E-05 | C |
| Example 10 | Perfusion | HEK293 | 43 | BlanCD | 120 | BlanCD | Yes | 375 | 1:1:1 | 3.125 | BEP | 1375 | 1.5 | 1 | | | | 7.7E-05 | C |
| Example 11 | Perfusion | HEK293 | 43 | BlanCD | 120 | BlanCD | Yes | 375 | 1:1:1 | 3.125 | BEP | 1375 | 4 | 1 | | | | 2.1E-06 | D |
| Example 12 | Perfusion | HEK293 | 43 | BlanCD | 120 | BlanCD | Yes | 60 | 1:1:1 | 0.5 | BEP | 1375 | 3 | 1 | | | | 2.5E-05 | A |
| Example 13 | Perfusion | HEK293 | 43 | BlanCD | 120 | BlanCD | Yes | 60 | 1:1:1 | 0.5 | BEP | 1375 | 6 | 1 | | | | 5.0E-05 | B |

(continued)

| | Preculture (cell supply) | | | | Electroporation | | | | | | | | | | | | | | |
|---|---|---|---|---|---|---|---|---|---|---|---|---|---|---|---|---|---|---|---|
| | | | | | Cell | | | Plasmid | | | EP method | Perforation pulse | | | Electrophoresis pulse | | | CNET product | CNET evaluation |
| | Culture method | Cell | Cell concentration | Culture medium | Cell concentration | Buffer | Medium exchange | Concentration C | Ratio | Nucleic acid/-cell | | Electric field Eh | Pulse width Th | Number of pulses Nh | Electric field El | Pulse width Tl | Number of pulses Nl | | |
| | - | - | Mcells ml | - | Mcell ml | - | - | ug ml | - | pg/cell | - | V/cm | ms | - | V/cm | ms | - | *1 | - |
| Example 14 | Perfusion | HEK293 | 43 | BlanCD | 120 | BlanCD | Yes | 150 | 3:1:1 | 1.25 | BEP | 1375 | 1.2 | 1 | | | | 2.5E-05 | A |
| Example 15 | Perfusion | HEK293 | 43 | BlanCD | 120 | BlanCD | Yes | 150 | 3:1:1 | 1.25 | BEP | 1375 | 2.4 | 1 | | | | 5.0E-05 | B |
| Example 16 | Perfusion | HEK293 | 43 | BlanCD | 120 | BlanCD | Yes | 150 | 1:1:1 | 1.25 | BEP | 1375 | 3.6 | 1 | | | | 7.4E-05 | C |
| Example 17 | Perfusion | HEK293 | 43 | BlanCD | 120 | BlanCD | Yes | 60 | 1:1:1 | 0.5 | BEP | 1375 | 1.2 | 1 | 500 | 5 | 1 | 2.5E-05 | A |
| Example 18 | Perfusion | HEK293 | 43 | BlanCD | 120 | BlanCD | Yes | 60 | 1:1:1 | 0.5 | BEP | 1375 | 1.2 | 1 | 500 | 10 | 1 | 4.0E-05 | B |
| Example 19 | Perfusion | HEK293 | 43 | BlanCD | 40 | BlanCD | Yes | 150 | 1:1:1 | 3.75 | FEP | 1375 | 3.6 | 1 | | | | 7.4E-05 | C |
| Example 20 | Perfusion | HEK293 | 43 | BlanCD | 40 | BlanCD | Yes | 150 | 1:1:1 | 3.75 | FEP | 1375 | 1.8 | 1 | | | | 3.7E-05 | A |
| Comparative Example 1 | Perfusion | HEK293 | 43 | BlanCD | 120 | BlanCD | Yes | 150 | 1:1:1 | 1.25 | BEP | 1375 | 0.02 | 1 | | | | 4.1E-03 | |
| Comparative Example 2 | Perfusion | HEK293 | 43 | BlanCD | 120 | BlanCD | Yes | 150 | 1:1:1 | 1.25 | BEP | 1375 | 10 | 6 | | | | 1.2E-07 | |

[Table 2]

| | Subsequent culture (AAV production culture) | | | Results | | | | |
|---|---|---|---|---|---|---|---|---|
| | Culture method | Cell concentration | Culture medium | Titer | Titer determination | Full ratio | Full ratio determination | Overall evaluation |
| | - | Mcells/ml | - | Vg/ml | - | % | - | - |
| Example 1 | Flask/shaking | 2 | BlanCD | 9.0E+09 | D | Not determined | Not determined | E |
| Example 2 | Flask/shaking | 2 | BlanCD | 3.2E+10 | C | *15* | C | C |
| Example 3 | Flask/shaking | 2 | BlanCD | 6.8E+10 | B | 51 | A | A |
| Example 4 | Flask/shaking | 2 | BlanCD | 1.6E+11 | A | 20 | B | B |
| Example 5 | Flask/shaking | 2 | BlanCD | 9.7E+10 | B | 8 | D | C |
| Example 6 | Flask/shaking | 2 | BlanCD | 2.8E+10 | C | 4 | E | D |
| Example 7 | Flask/shaking | 2 | BlanCD | 5.1E+10 | B | 14 | C | C |
| Example 8 | Flask/shaking | 2 | BlanCD | 1.1E+11 | A | 39 | A | A |
| Example 9 | Flask/shaking | 2 | BlanCD | 2.1E+11 | A | 18 | C | C |
| Example 10 | Flask/shaking | 2 | BlanCD | 1.6E+11 | A | 9 | D | C |
| Example 11 | Flask/shaking | 2 | BlanCD | 3.5E+10 | C | 3 | E | D |
| Example 12 | Flask/shaking | 2 | BlanCD | 2.6E+10 | C | 31 | A | A |
| Example 13 | Flask/shaking | 2 | BlanCD | 5.2E+10 | B | 5 | D | C |
| Example 14 | Flask/shaking | 2 | BlanCD | 4.7E+10 | C | 59 | A | A |
| Example 15 | Flask/shaking | 2 | BlanCD | 9.0E+10 | B | 23 | B | B |
| Example 16 | Flask/shaking | 2 | BlanCD | 7.0E+10 | B | 8 | D | C |
| Example 17 | Flask/shaking | 2 | BlanCD | 2.1E+11 | A | 33 | A | A |
| Example 18 | Flask/shaking | 2 | BlanCD | 4.4E+11 | A | 11 | C | C |
| Example 19 | Flask/shaking | 2 | BlanCD | 1.7E+11 | A | 16 | C | C |
| Example 20 | Flask/shaking | 2 | BlanCD | 1.0E+11 | A | 31 | A | A |
| Comparative Example 1 | Flask/shaking | 2 | BlanCD | <1E9 | E | - | × | F |
| Comparative Example 2 | Flask/shaking | 2 | BlanCD | <1E9 | E | Not determined | × | F |

*1 μg/ml·V/cm·ms

Explanation of References

10: upper electrode holding plate

20: lower electrode holding plate

30: flow channel plate

32: opening portion (flow channel)

41: electrode pair

41a: upper electrode

41b: lower electrode

50: inflow port

52: outflow port

100: electroporation device

al: front surface portion of upper electrode

a2: rear surface portion of upper electrode

b1: front surface portion of lower electrode

b2: rear surface portion of lower electrode

L: electrode length

W: electrode width (flow channel width)

D: inter-electrode distance (gap)

[Sequence list] International application 22F01305W1JP24012393_32.xml based on International Patent Cooperation Treaty

## Claims

**1.** A virus production method comprising:

a nucleic acid introduction step of introducing a nucleic acid into cells via electroporation to obtain cells into which the nucleic acid has been introduced; and
a culture step of culturing the cells into which the nucleic acid has been introduced,
wherein a CNET product represented by the following formula in the electroporation is $1 \times 10^4$ or more and $1 \times 10^7$ or less,

$$\sum_{k=1}^{n} C|N_k E_k T_k| \quad \text{or} \quad \sum_{k=1}^{n} C|N_k E_k T_k| + \sum_{l=1}^{m} C|N_l E_l T_l|$$

in the formulae, C represents a nucleic acid concentration in terms of μg/mL,
$N_k$ represents the number of pulses, and $N_k$ is an integer of 1 or more,
$E_k$ represents a pulsed electric field in terms of V/cm, and $E_k$ is 500 V/cm or more and 2,000 V/cm or less,
$T_k$ represents a pulse duration in terms of ms,
$N_l$ represents the number of pulses, and $N_l$ is an integer of 1 or more,
$E_l$ represents a pulsed electric field in terms of V/cm, and $E_l$ is 50 V/cm or more and less than 500 V/cm,
$T_l$ represents a pulse duration in terms of ms,
k represents an integer from 1 to n,
l represents an integer from 1 to m,
n represents an integer of 1 or more, and
m represents an integer of 1 or more.

**2.** The virus production method according to claim 1,
wherein the introduction of the nucleic acid is performed only via the electroporation.

**3.** The virus production method according to claim 1 or 2,
wherein the CNET product is $1 \times 10^5$ or more and $1 \times 10^6$ or less.

**4.** The virus production method according to any one of claims 1 to 3,

wherein the CNET product is represented by $\sum_{k=1}^{n} C|N_k E_k T_k| + \sum_{l=1}^{m} C|N_l E_l T_l|$, and

$$\sum_{k=1}^{n} C|N_k E_k T_k| < \sum_{l=1}^{m} C|N_l E_l T_l|$$ is satisfied.

**5.** The virus production method according to any one of claims 1 to 4,
wherein the nucleic acid concentration C is 10 to 500 μg/mL.

**6.** The virus production method according to any one of claims 1 to 5,
wherein in a virus produced in the culture step, a ratio of an amount of capsids containing a full-length gene to a total amount of capsids is 30% or more.

**7.** The virus production method according to any one of claims 1 to 6,
wherein the nucleic acid is introduced into the cells in an absence of a transfection reagent.

**8.** The virus production method according to any one of claims 1 to 7,

wherein the cells are in a suspension in the nucleic acid introduction step, and
the suspension has a conductivity of 5 to 20 mS/cm.

**9.** The virus production method according to any one of claims 1 to 8,

wherein the cells are in a suspension in the nucleic acid introduction step and the culture step, and
the suspension is a culture medium.

**10.** The virus production method according to any one of claims 1 to 9,
wherein after the nucleic acid introduction step, an additional nucleic acid introduction is not performed.

**11.** The virus production method according to any one of claims 1 to 10,
wherein a virus is an adeno-associated virus.

**12.** The virus production method according to any one of claims 1 to 11,
wherein the nucleic acid contains one or more of an adeno-associated virus gene and a virus helper gene.

**13.** The virus production method according to any one of claims 1 to 12, wherein the nucleic acid contains at least one or more virus helper genes.

**14.** The virus production method according to any one of claims 1 to 13, wherein the nucleic acid contains four or more genes selected from the group consisting of a gene for treatment or prevention, a Rep gene, a Cap gene, an E2 gene, an E4 gene, and a VA-RNA1 gene.

**15.** The virus production method according to any one of claims 1 to 14, wherein the nucleic acid is introduced into the cells using a plurality of plasmids.

**16.** The virus production method according to any one of claims 1 to 15, wherein the electroporation is flow electroporation.

**17.** The virus production method according to any one of claims 1 to 16, wherein a cell concentration during the nucleic acid introduction is $10 \times 10^6$ cells/mL to $200 \times 10^6$ cells/mL.

**18.** The virus production method according to any one of claims 1 to 17, wherein feeding of liquid is performed aseptically in the nucleic acid introduction step and the culture step.

**19.** The virus production method according to any one of claims 1 to 18, wherein the cells are animal cells.

**20.** The virus production method according to any one of claims 1 to 19, wherein the cells are HEK cells.

**21.** The virus production method according to any one of claims 1 to 20, further comprising: recovering a produced virus.

FIG. 1

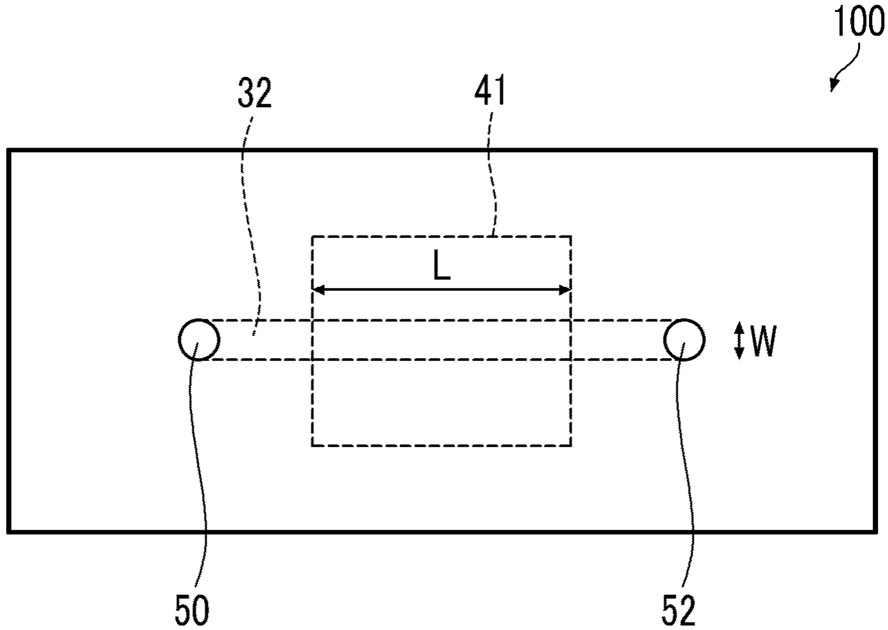
100
32
41
L
W
50
52

FIG. 2

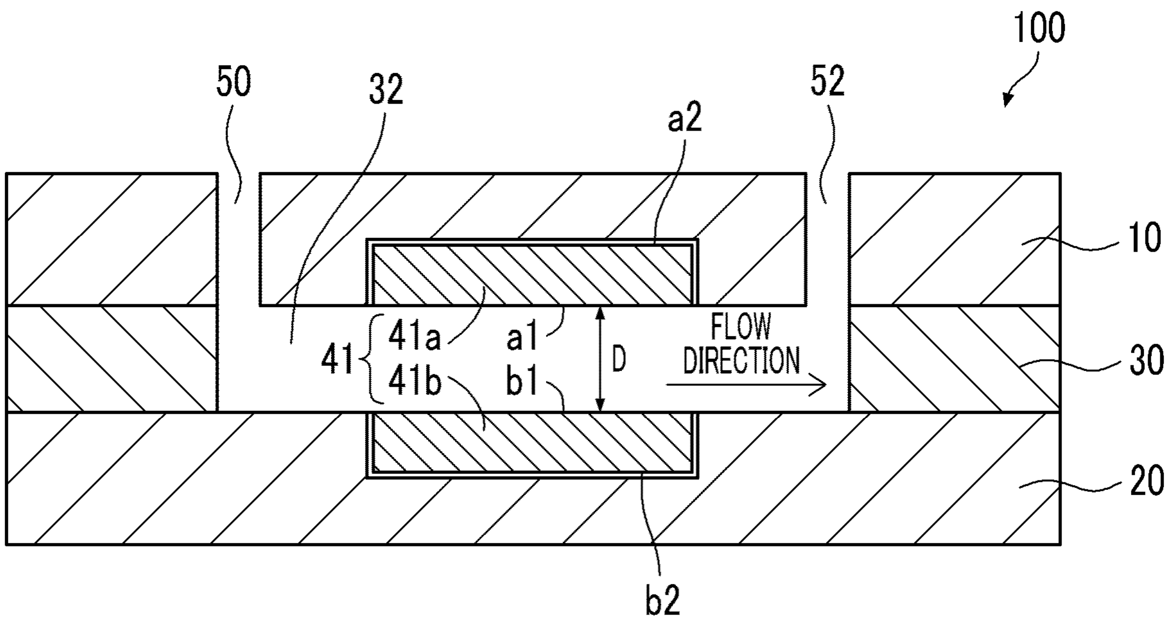
100
50
32
52
a2
10
41
41a
41b
a1
b1
D
FLOW DIRECTION
30
20
b2

FIG. 3

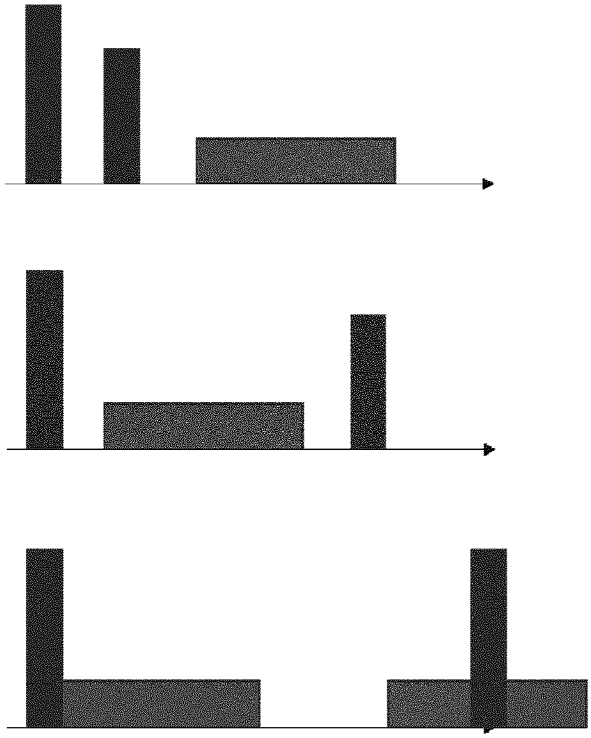

FIG. 4

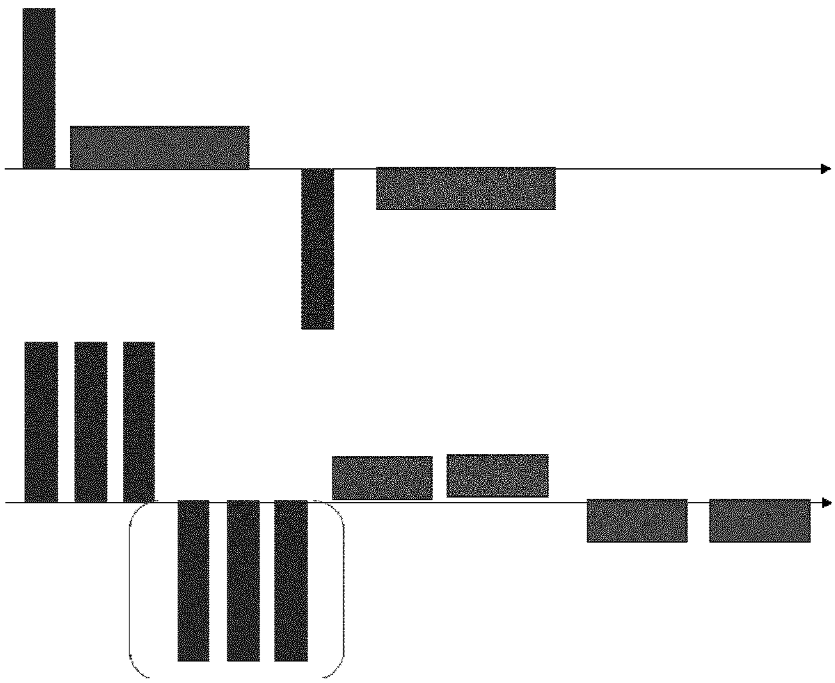

FIG. 5

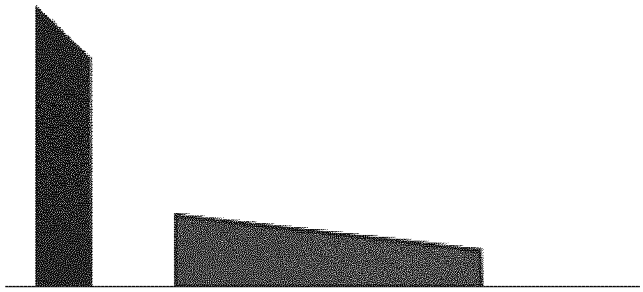

FIG. 6

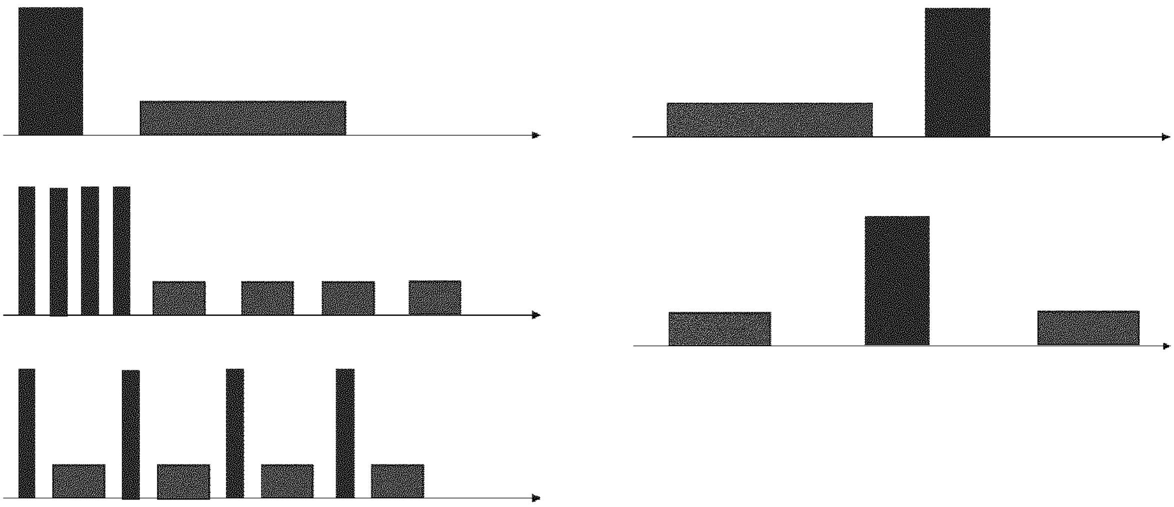

FIG. 7

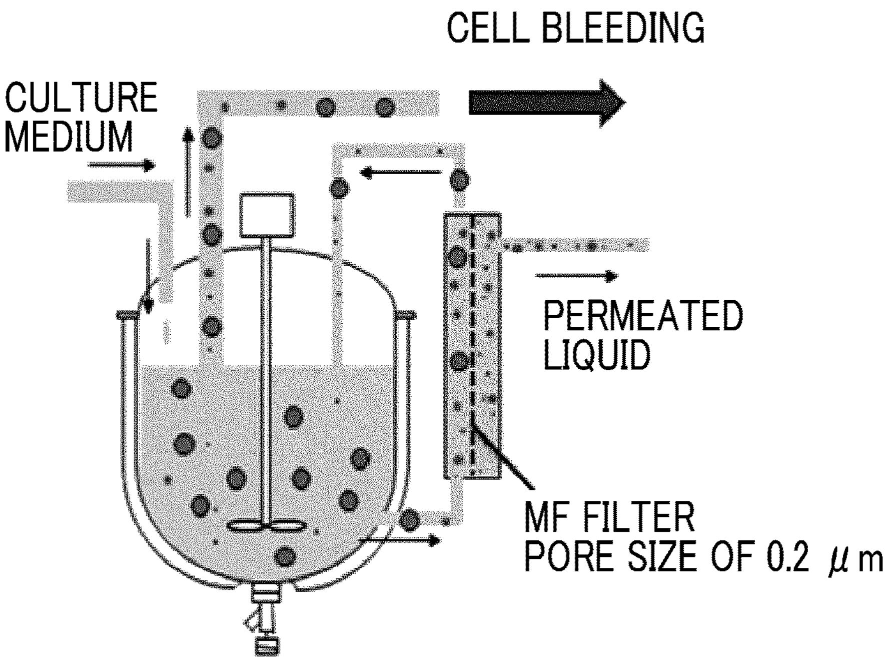
CELL BLEEDING
CULTURE MEDIUM
PERMEATED LIQUID
MF FILTER
PORE SIZE OF 0.2 μm

FIG. 8

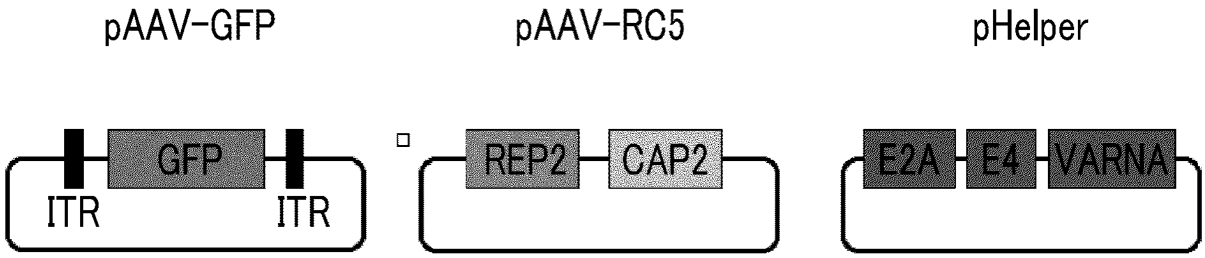
pAAV-GFP
pAAV-RC5
pHelper
GFP
ITR
ITR
REP2
CAP2
E2A
E4
VARNA

FIG. 9

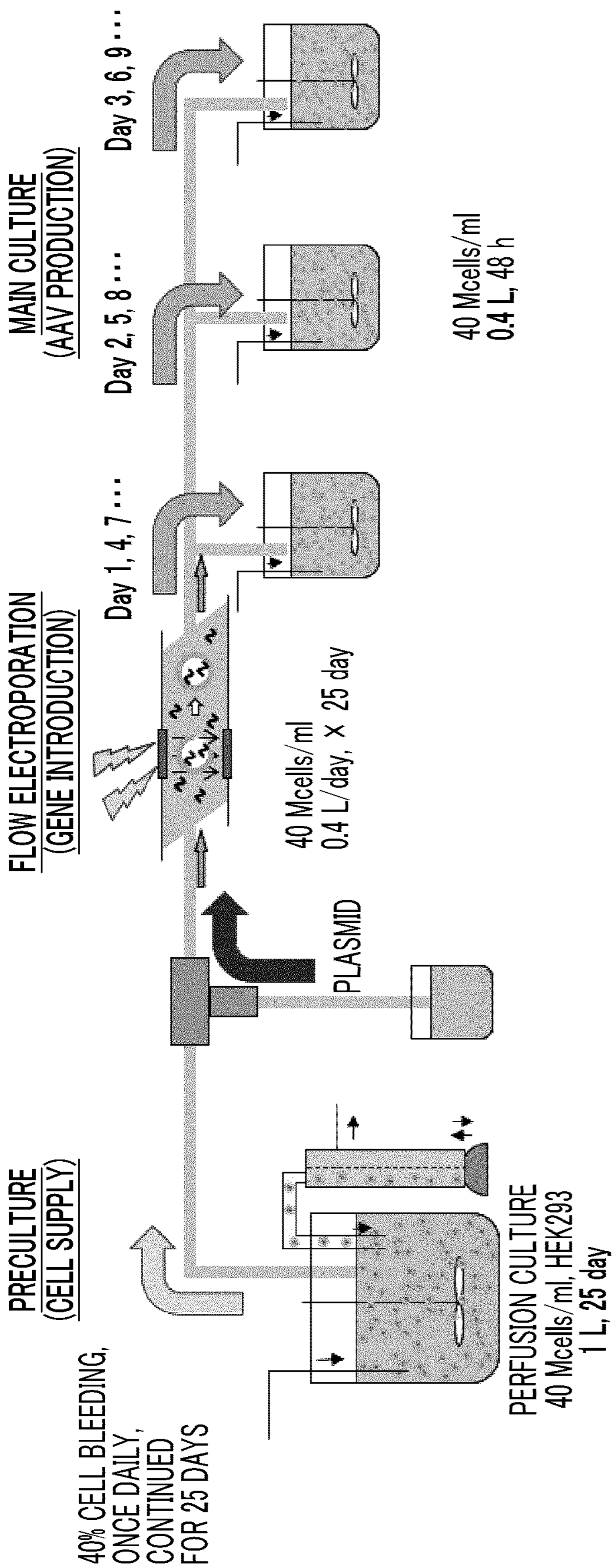
PRECULTURE
(CELL SUPPLY)
40% CELL BLEEDING,
ONCE DAILY,
CONTINUED
FOR 25 DAYS
PERFUSION CULTURE
40 Mcells/ml, HEK293
1 L, 25 day
PLASMID
FLOW ELECTROPORATION
(GENE INTRODUCTION)
40 Mcells/ml
0.4 L/day, × 25 day
MAIN CULTURE
(AAV PRODUCTION)
Day 1, 4, 7 . . .
Day 2, 5, 8 . . .
Day 3, 6, 9 . . .
40 Mcells/ml
0.4 L, 48 h

## INTERNATIONAL SEARCH REPORT

International application No.
**PCT/JP2024/012393**

**A. CLASSIFICATION OF SUBJECT MATTER**

***C12N 15/86***(2006.01)i; ***C12N 5/10***(2006.01)i; ***C12N 7/00***(2006.01)i; ***C12N 7/02***(2006.01)i; ***C12N 13/00***(2006.01)i; ***C12N 15/63***(2006.01)i; ***C12N 15/87***(2006.01)i; ***C12N 15/864***(2006.01)i
FI: C12N15/86 Z; C12N5/10; C12N15/864 Z; C12N15/63 Z; C12N7/02 ZNA; C12N15/87; C12N13/00; C12N7/00 ZNA

According to International Patent Classification (IPC) or to both national classification and IPC

**B. FIELDS SEARCHED**

Minimum documentation searched (classification system followed by classification symbols)

C12N15/86; C12N5/10; C12N7/00; C12N7/02; C12N13/00; C12N15/63; C12N15/87; C12N15/864

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); MEDLINE (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2021-535738 A (NANOCAV LLC) 23 December 2021 (2021-12-23) claims 1-54, paragraphs [0132], [0144], examples 2, 6, tables 3, 5, 6, 8 | 1-3, 5-10, 14-21 |
| Y | | 1-3, 5-21 |
| A | | 4 |
| Y | MAXWELL, F., et al., "Improved production of recombinant AAV by transient transfection of NB324K cells using electroporation.", JOURNAL OF VIROLOGICAL METHODS, January 1997, vol. 63, no. 1-2, pp. 129-136, DOI: 10.1016/s0166-0934(96)02121-0 abstract, p. 131, fig. 3 | 1-3, 5-21 |
| A | | 4 |
| A | JP 11-502133 A (ENTRE MED, INC.) 23 February 1999 (1999-02-23) claims 1-11 | 1-21 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 June 2024** | **25 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**

International application No.
**PCT/JP2024/012393**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/012393**

| Patent document cited in search report | Publication date (day/month/year) | Patent family member(s) | Publication date (day/month/year) |
|---|---|---|---|
| JP 2021-535738 A | 23 December 2021 | WO 2021/007315 A1<br>claims 1-54, paragraphs [00137], [00148], examples 2, 6, tables 3, 5, 6, 8<br>US 2020/0340014 A1<br>EP 3818143 A1<br>KR 10-2021-0028687 A<br>CN 112639112 A | |
| JP 11-502133 A | 23 February 1999 | WO 1996/028199 A1<br>claims 1-11<br>US 5720921 A<br>EP 814855 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020511153 A
- WO 2018159847 A
- WO 2019049843 A
- WO 2019181234 A
- WO 2019239780 A
- WO 2020003833 A
- WO 2020162125 A
- WO 2021187008 A
- WO 2022196710 A
- WO 2023054556 A
- WO 2015005430 A
- WO 2022224803 A
- WO 2023157673 A
- WO 2023223931 A
- JP 24012393 B